# EUROPEAN PATENT SPECIFICATION

(11) **EP 4 378 383 B1**
(45) Date of publication and mention of the grant of the patent: **27.05.2026**
(21) Application number: 22860382.5
(22) Date of filing: 18.08.2022
(51) Int. Cl.: A61B 5/022

(54) **ELECTRONIC DEVICE**
ELEKTRONISCHE VORRICHTUNG
DISPOSITIF ÉLECTRONIQUE

(30) Priority: 26.08.2021 CN 202122040209 U; 16.02.2022 CN 202210141000
(43) Date of publication of application: 05.06.2024
(73) Proprietor: Huawei Technologies Co., Ltd., Shenzhen, Guangdong 518129 (CN)
(72) Inventor: LIN, Zhongwei, Shenzhen, Guangdong 518129 (CN); JIN, Junye, Shenzhen, Guangdong 518129 (CN); ZHENG, Wenhui, Shenzhen, Guangdong 518129 (CN)
(74) Representative: Huawei European IPR
(86) International application number: PCT/CN2022/113346
(87) International publication number: WO 2023/025036

(56) References cited:
- CN-A- 112 826 477
- CN-U- 207 024 050
- CN-U- 207 780 519
- CN-U- 209 404 771
- JP-A- 2004 135 779
- KR-A- 20160 121 307
- US-A1- 2016 223 992
- US-A1- 2020 085 319
- US-A1- 2020 272 106

## Description

This application claims priorities to Chinese Patent Application No. 202122040209.9, filed with the China National Intellectual Property Administration on August 26, 2021 and entitled "ELECTRONIC DEVICE", and to Chinese Patent Application No. 202210141000.8, filed with the China National Intellectual Property Administration on February 16, 2022 and entitled "ELECTRONIC DEVICE".

### TECHNICAL FIELD

This application relates to the field of electronic device technologies, and in particular, to an electronic device.

### BACKGROUND

Electronic devices for measuring blood pressure (for example, a blood pressure watch or a blood pressure wrist strap) have smaller sizes and lighter weight, and therefore, the electronic devices can be worn by a user for a long time, meeting a requirement for long-term and real-time blood pressure detection. A basic principle of measuring blood pressure by the blood pressure watch is as follows: When the blood pressure watch is worn on a wrist of the user, an airbag on a strap of the blood pressure watch is closely attached to a radial artery of the user, and it may be considered that a pressure value in the airbag is approximately equal to a pressure value of the radial artery. The blood pressure watch may determine a blood pressure value of the user by detecting the pressure value in the airbag. The blood pressure value may include systolic blood pressure (systolic blood pressure, SBP) and diastolic blood pressure (diastolic blood pressure, DBP).

Generally, the blood pressure watch includes a watch body and an airbag strap. However, the user may assemble an incorrect airbag strap to the watch body, affecting measurement accuracy of the blood pressure watch.
US2020085319 discloses a wearable device capable of blood pressure measurement, comprising a wrist band assembly, a display unit, and a detachable bladder, wherein the wrist band assembly is provided with a micro air pump connected to the detachable bladder, an air pressure sensor provided on a side of the detachable bladder, and a processor connected to the micro air pump and the air pressure sensor, and the processor activates the micro air pump according to a trigger signal in order for the micro air pump to inflate the detachable bladder and for a user's blood pressure parameters to be derived from data sent by the air pressure sensor to the processor.
US2020272106 disclose an electronic device can detect an identification of the band, which can serve as an input to initiate actions performed by the electronic device. For example, a type, model, color, size, or other characteristic of a band can be determined and used to select a corresponding action performed by the electronic device.
US2016223992 disclose a wearable device includes a first strap portion attached or detached from a body portion and provided to allow the body portion to be worn on a human body, a second strap portion provided in the body portion to be exchanged with the first strap portion, electrically connected to the body portion, and having at least one member to provide a signal generated from the members to the body portion, and a connecting portion electrically connecting the body portion with the first strap portion or electrically connecting the body portion with the second strap portion.

### SUMMARY

The invention is defined in the appended claims. Embodiments of this application provide an electronic device, to improve accuracy of blood pressure measurement.

According to a first aspect, an electronic device is provided. The electronic device includes a watch body, a first strap, and a second strap. The watch body includes a Hall effect sensor and a processor. A first airbag and a first magnet are disposed on the first strap, and a first magnetic pole of the first magnet is disposed close to a mounting part of the first strap. A second airbag and a second magnet are disposed on the second strap, or the second strap includes a cover and a second magnet, and a second magnetic pole of the second magnet is disposed close to a mounting part of the second strap. The second magnetic pole is different from the first magnetic pole, a length of the first airbag is different from a length of the second airbag, and the mounting part of the first strap or the mounting part of the second strap is configured to be detachably connected to the watch body. The processor is configured to: if the Hall effect sensor detects a magnetic field of the first magnetic pole, determine that the first strap is disposed on the watch body; or if the Hall effect sensor detects a magnetic field of the second magnetic pole, determine that the second strap is disposed on the watch body.

It may be understood that either of the first strap and the second strap is disposed on the watch body. To be specific, one watch body matches one strap. A user may choose to dispose the first strap or the second strap on the watch body as required.

In this application, the Hall effect sensor is disposed on the watch body, and the magnet is disposed on the airbag. Magnetic poles that are of magnets disposed on airbags of different types of straps and that face the Hall effect sensor are different, so that the Hall effect sensor can obtain, by correspondingly identifying a detected type of a magnetic pole, a type of a strap disposed on the watch body. The user can learn, based on an identification result, whether the user wears an incorrect strap, thereby avoiding a problem that blood pressure precision decreases because the user uses the incorrect strap.

In a possible implementation, the watch body further includes an air pump and a communication hole. The air pump communicates with the communication hole, and the second airbag or the cover includes an air nozzle. When the second strap is disposed on the watch body, the air nozzle communicates with the communication hole. The processor is further configured to: if the second strap is disposed on the watch body, drive the air pump to inflate the second strap, to obtain test data; and compare the test data with preset data, to determine a type of the test data. If the test data is first data, the second airbag is disposed on the second strap. If the test data is second data, the second strap includes the cover.

It may be understood that, when the second airbag is disposed on the second strap, the second strap has an airbag. When the second strap includes the cover, the second strap does not have an airbag. Therefore, when there is an airbag or there is no airbag in an inflated state, atmospheric pressure changes identified by a pressure sensor are different. Because the second strap without the airbag cannot store gas, a pressurization curve slope rapidly increases in a short time according to a fixed slope, and a pressurization curve slope of the second strap with the airbag slowly increases in a period of time according to a fixed slope.

Pressurization curve slopes of the second strap with the airbag and the second strap without the airbag in the inflated state are different. The pressurization curve slope is the test data. A pressurization curve slope of the second strap is compared with preset data (the pressurization curve slope with the airbag and the pressurization curve slope without the airbag). If the pressurization curve slope of the second strap matches the pressurization curve slope with the airbag, it indicates that the test data is the first data, and the second airbag is disposed on the second strap. If the pressurization curve slope of the second strap matches the pressurization curve slope without the airbag, it indicates that the test data is the second data, and the second strap includes the cover.

In this implementation, the Hall effect sensor is disposed on the watch body, and the magnet is disposed on the airbag or the cover. Magnetic poles that are of magnets disposed on airbags or covers of the first strap and the second strap and that face the Hall effect sensor are different, so that the Hall effect sensor can obtain, by correspondingly identifying a detected type of a magnetic pole, whether the strap disposed on the watch body is the first strap or the second strap. If the strap is the second strap, the second strap is further inflated to identify whether the second strap has the second airbag. The processor presents an identification result to the user by using a display, and the user may learn, based on the identification result, whether the user wears an incorrect strap, thereby avoiding a problem that blood pressure precision decreases because the user uses the incorrect strap.

Certainly, in another implementation, when the Hall effect sensor identifies a magnetic pole of a strap, the air pump in the watch body may inflate the strap, to determine, in a short time, whether the strap is the strap without the second airbag. Therefore, the processor may quickly determine a type of the strap in the short time based on a joint detection result of how the Hall effect sensor and the magnetic pole collaborate and how to inflate the strap.

In a possible implementation, the first strap is applicable to a user whose wrist circumference ranges from 130 mm to 160 mm (including 130 mm and 160 mm), and the second strap is applicable to a user whose wrist circumference ranges from 161 mm to 230 mm (including 161 mm and 230 mm). Users with different wrist circumferences may select straps with different airbag lengths as required. On the premise that an airbag of a strap can fully cover a radial artery and an ulnar artery, it can be further ensured that a length of the strap is not excessively long, thereby ensuring user experience.

In a possible implementation, the watch body further includes the air pump. The processor can further control the air pump to inflate the first strap or the second strap, to obtain test data, and detect that the first strap or the second strap is disposed on the watch body based on the test data. In this identification manner, the Hall effect sensor or the magnet may not be disposed on the electronic device, so that components of the electronic device can be reduced, costs can be reduced, and integration of the electronic device can be improved. However, identification time of identifying the strap by inflating the strap is longer compared with identification time of first identifying the first strap and the second strap by collaboration between the Hall effect sensor and the magnet, and then further identifying a long airbag strap and a common strap by inflating the strap, and user experience is not as good as user experience of the latter.

In a possible implementation, the electronic device further includes an obtaining module and a comparison module. The obtaining module is configured to obtain wrist circumference data of a user, and the comparison module is configured to compare the wrist circumference data with a type of a strap, to detect that the strap is selected correctly or incorrectly. In this implementation, the type of the strap is identified, and the type of the strap is compared with the wrist circumference data of the user, to determine that the user selects a strap for installation correctly or incorrectly. If the strap is correctly selected, blood pressure measurement is performed. If the strap is incorrectly selected, the user is reminded to replace the strap with a correct strap. In this way, the user is prevented from selecting an incorrect strap, and blood pressure measurement is more accurate.

In a possible implementation, the processor is further configured to: if the strap is selected incorrectly, remind the user to replace the strap with a correct strap. The user is prevented from selecting an incorrect strap, so that blood pressure measurement is more accurate.

In a possible implementation, the watch body further includes a touchscreen or a button. The touchscreen or the button is configured to receive an input operation of the user, and the obtaining module is configured to obtain an electrical signal of the touchscreen or the button, to obtain the wrist circumference data input by the user. Certainly, in another implementation, the obtaining module may further obtain the wrist circumference data of the user through automatic measurement.

In a possible implementation, the processor is further configured to: if the Hall effect sensor detects that there is no magnetic field, determine that an airbag or the cover is not disposed on the watch body. It may be understood that, a case that the Hall effect sensor detects that there is no magnetic field may alternatively be a case that the airbag is loosely disposed. When receiving that the airbag is not disposed on the watch body of the electronic device, the user may dispose the airbag on the watch body or check whether the airbag is loose, to avoid that the user washes hands or takes a shower to cause water damage to the device when the airbag is loose.

In a possible implementation, the second strap includes a first strap body and a second strap body. The first strap body and the second strap body are respectively connected to two sides of the watch body. The second airbag is disposed on an inner side of the first strap body, and the second magnet is disposed on a part that is of the second airbag and that is close to the watch body. In other words, the airbag is disposed on only either of the strap bodies, and does not block the watch body, so that the electronic device may dispose a sensing component on the watch body, to detect a heart rate of the user.

In a possible implementation, the second strap includes a first strap body and a second strap body. The first strap body and the second strap body are respectively connected to two sides of the watch body. The second airbag is disposed on inner sides of the first strap body and the watch body, and the second magnet is disposed on a part that is of the second airbag and that is close to the watch body. In other words, the airbag blocks either of the strap bodies and the watch body.

In a possible implementation, the second strap includes a first strap body and a second strap body. The first strap body and the second strap body are respectively connected to two sides of the watch body. The second airbag is disposed on inner sides of the first strap body, the watch body, and the second strap body, and the second magnet is disposed on a part that is of the second airbag and that is close to the watch body.

In a possible implementation, the second strap includes a first strap body and a second strap body. The first strap body and the second strap body are respectively connected to two sides of the watch body. The cover is connected to the watch body, and the second magnet is disposed on the cover. When the user does not need to measure blood pressure, the user may detach the airbag from the strap, and then cover a vent hole of the watch body with the cover, to ensure sealing of the watch body and prevent the electronic device from being damaged.

### BRIEF DESCRIPTION OF DRAWINGS

To describe the technical solutions in embodiments of this application or the background more clearly, the following describes the accompanying drawings used in embodiments of this application or the background.
FIG. 1 is a schematic diagram of a structure of an electronic device in a first embodiment according to an embodiment of this application;
FIG. 2 is a schematic diagram of a structure of the electronic device shown in FIG. 1 from another angle;
FIG. 3 is a schematic diagram of a structure of a watch body of the electronic device shown in FIG. 2;
FIG. 4 is a schematic sectional view of a structure of the electronic device shown in FIG. 2;
FIG. 5 is a schematic exploded view of a structure of the electronic device shown in FIG. 2;
FIG. 6 is a schematic diagram of a structure of a first strap body of the electronic device shown in FIG. 5 from another angle;
FIG. 7 is a schematic diagram of a plurality of types of first strap bodies of the structure shown in FIG. 2;
FIG. 8 is a schematic diagram of a structure of a Hall effect sensor of the structure shown in FIG. 5;
FIG. 9 is a schematic diagram of a plurality of types of first strap bodies of the structure shown in FIG. 2 according to another embodiment;
FIG. 10 is a schematic diagram of a partial structure of the electronic device shown in FIG. 2 according to another embodiment;
FIG. 11 is a schematic diagram of a structure of the electronic device shown in FIG. 2 according to another embodiment;
FIG. 12 is a schematic diagram of a structure of the electronic device shown in FIG. 2 according to another embodiment;
FIG. 13 is a schematic flowchart of a strap identification method of an electronic device according to a first embodiment;
FIG. 14 is a schematic diagram of identification of the strap identification method shown in FIG. 13;
FIG. 15 is a schematic diagram of a user interface of the strap identification method shown in FIG. 13;
FIG. 16 is a schematic diagram of a user interface of the strap identification method shown in FIG. 13;
FIG. 17 is a schematic diagram of a user interface of the strap identification method shown in FIG. 13;
FIG. 18 is a schematic flowchart of a strap identification method of an electronic device according to a second embodiment;
FIG. 19 is a schematic diagram of identification of the strap identification method shown in FIG. 18;
FIG. 20 is a schematic diagram of pressurization curves of a common strap and a long airbag strap;
FIG. 21 is a schematic diagram of a user interface of the strap identification method shown in FIG. 18;
FIG. 22 is a schematic flowchart of a strap identification method of an electronic device according to a third embodiment;
FIG. 23 is a schematic diagram of identification of the strap identification method shown in FIG. 22;
FIG. 24 is a schematic diagram of a user interface of the strap identification method shown in FIG. 22;
FIG. 25 is a schematic diagram of a user interface of the strap identification method shown in FIG. 22;
FIG. 26 is a schematic diagram of a user interface of the strap identification method shown in FIG. 22;
FIG. 27 is a schematic diagram of a user interface of the strap identification method shown in FIG. 22;
FIG. 28 is a schematic diagram of a user interface of the strap identification method shown in FIG. 22;
FIG. 29 is a schematic diagram of a user interface of the strap identification method shown in FIG. 22;
FIG. 30 is a schematic diagram of a user interface of the strap identification method shown in FIG. 22; and
FIG. 31 is a schematic diagram of a user interface of the strap identification method shown in FIG. 22.

### DESCRIPTION OF EMBODIMENTS

The following describes embodiments of this application with reference to the accompanying drawings in embodiments of this application.

In the descriptions of embodiments of this application, it should be noted that terms "dispose" and "connection" should be understood in a broad sense unless there is a clear stipulation and limitation. For example, "connection" may be a detachable connection, an un-detachable connection, a direct connection, or an indirect connection through an intermediate medium. "Fixed connection" means a connection to each other and a relative position relationship unchanged after the connection. The orientation terms mentioned in embodiments of this application, for example, "inside" and "outside", are merely directions with reference to the accompanying drawings. Therefore, the orientation terms are used to better and more clearly describe and understand embodiments of this application, instead of indicating or implying that a specified apparatus or element should have a specific orientation, and be constructed and operated in the specific orientation. Therefore, the orientation terms cannot be understood as a limitation on embodiments of this application. "A plurality of" means at least two.

It may be understood that the specific embodiments described herein are merely used to explain a related application, but are not intended to limit this application. In addition, it should be further noted that, for ease of description, only a part related to this application is shown in the accompanying drawings.

It should be noted that embodiments in this application and the features in embodiments may be mutually combined in the case of no conflict. This application is described in detail in the following with reference to the accompanying drawings by using embodiments.

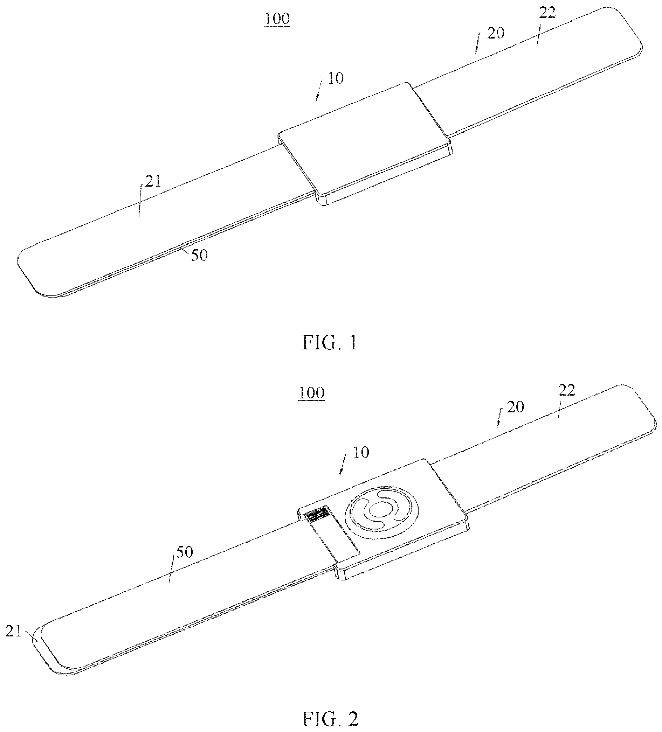

FIG. 1 is a schematic diagram of a structure of an electronic device in a first embodiment according to an embodiment of this application, and FIG. 2 is a schematic diagram of a structure of the electronic device shown in FIG. 1 from another angle. FIG. 1 is a schematic front view of the electronic device, and FIG. 2 is a schematic rear view of the structure of the electronic device.

An electronic device 100 includes but is not limited to a watch, a smart watch, a band, a smart band, and another wrist electronic product. In the embodiment shown in FIG. 1, an example in which the electronic device 100 is the smart watch is used for description.

The electronic device 100 includes a watch body 10 and a strap 20. The strap 20 is disposed on the watch body 10, and an airbag 50 is disposed on the strap 20. The airbag 50 is disposed on an inner side of the strap 20 and is connected to the watch body 10. In this embodiment, the airbag 50 is detachably connected to the strap 20 and the watch body 10, so that the airbag 50 may be detached from the strap 20 and the watch body 10 when blood pressure measurement is not performed, and a user feels more comfortable in daily wear. Certainly, in another embodiment, the airbag may be detachably connected to the watch body 10, and is fastened to the strap. Alternatively, the airbag may be fastened to the watch body and the strap.

There are two straps 20, and the two straps 20 are a first strap body 21 and a second strap body 22. The first strap body 21 and the second strap body 22 are respectively connected to two opposite sides of the watch body 10. In this embodiment, both the first strap body 21 and the second strap body 22 are detachably connected to the two opposite sides of the watch body 10. Therefore, it is convenient to replace different types of straps for the watch body 10, to meet various wearing requirements of the user, and improve user experience. Certainly, in another embodiment, the first strap body 21 and the second strap body 22 may alternatively be fastened to the two opposite sides of the watch body 10.

For example, a mounting part and a first lock part (not shown in the figure) are disposed on the first strap body 21, and a mounting part and a second lock part (not shown in the figure) are disposed on the second strap body 22. The mounting part of the first strap body 21 and the mounting part of the second strap body 22 are respectively disposed on the two opposite sides of the watch body 10. The first lock part and the second lock part are detachably locked and held by each other, to wear the electronic device 100 on a wrist of the user. It should be understood that, a mating structure between the first lock part and the second lock part may be a watch buckle structure, for example, a hook buckle, a hidden clasp, a butterfly clasp, a leather deployment buckle, a folding clasp with safety, a foldover clasp, or a Tang buckle. This is not limited in this application.

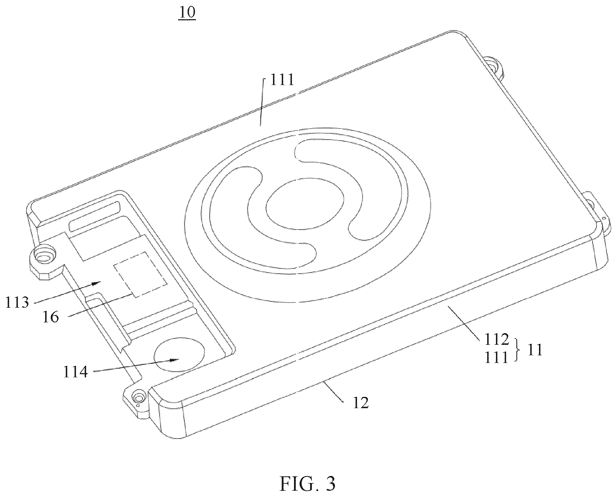

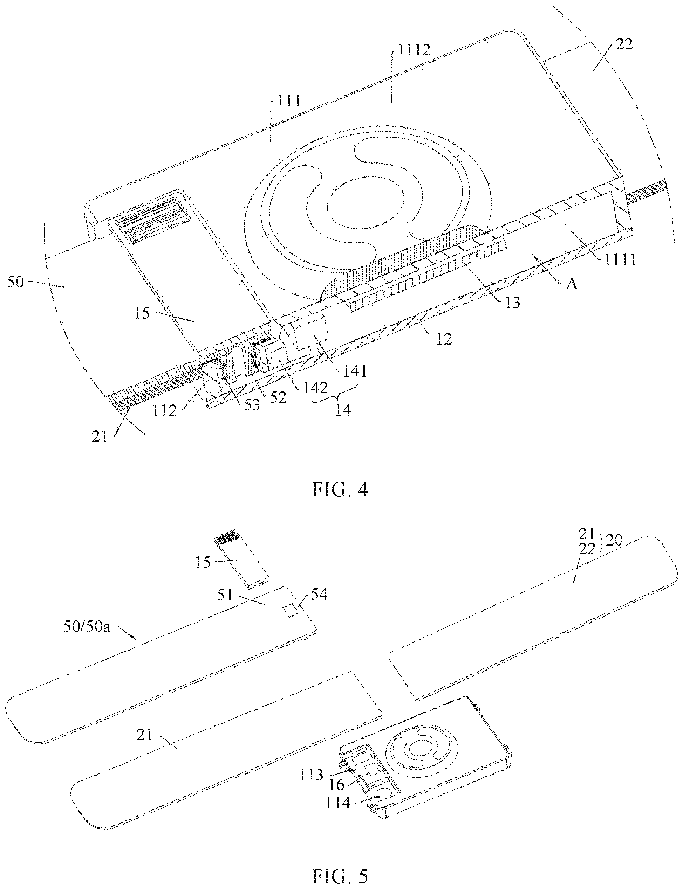

FIG. 3 is a schematic diagram of a structure of a watch body of the electronic device shown in FIG. 2, and FIG. 4 is a schematic sectional view of the electronic device shown in FIG. 2.

In this embodiment, the watch body 10 is of a cuboid structure, and the watch body 10 includes a housing 11, a display 12, a processor 13, and a blood pressure measurement component 14. The display 12 is connected to the housing 11, and accommodation space A of the watch body 10 is formed by surrounding the display 12 and the housing 11. Both the processor 13 and the blood pressure measurement component 14 are located in the accommodation space A. The cuboid structure described above includes the cuboid structure and a structure approximate to a cuboid. The structure approximate to the cuboid means that an outer surface of the cuboid may be partially concave or partially convex. The following descriptions of a structure shape may be understood in a same way. It should be understood that, in another embodiment, the watch body 10 may alternatively be of a cylindrical structure, a frustum structure, a cubic structure, or another special-shaped structure.

The housing 11 includes a bottom wall 111 and a frame 112. The bottom wall 111 is fastened to one side of the frame 112, and the display 12 is fastened to one side that is of the frame 112 and that is away from the bottom wall 111. In other words, the bottom wall 111 and the display 12 are respectively fastened to two opposite sides of the frame 112. The bottom wall 111, the frame 112, and the display 12 jointly surround the accommodation space A of the watch body 10. When the user wears the electronic device 100, the display 12 is disposed away from the wrist of the user, and the bottom wall 111 is disposed close to the wrist of the user.

In this embodiment, the bottom wall 111 may be detachably disposed on the frame 112, to maintain and replace functional components such as a memory card, a SIM card, and a speaker in the watch body 10. Certainly, in another embodiment, the bottom wall 111 and the frame 112 may alternatively be an integral structure, to improve stability of the watch body 10.

The frame includes a first side and a second side that are disposed opposite to each other, and the mounting part of the first strap body 21 and the mounting part of the second strap body 22 are detachably disposed on the first side and the second side respectively.

The bottom wall 111 includes a top surface 1111 facing the accommodation space A and a bottom surface 1112 disposed opposite to the top surface 1111. A mounting groove 113 is provided on the bottom surface 1112, and the mounting groove 113 is configured to dispose the airbag 50. In this embodiment, there is one mounting groove 113, and the mounting groove 113 is provided close to the first side. Certainly, in another embodiment, two opposite sides of the mounting groove 113 may be respectively close to the first side and the second side. Alternatively, the mounting groove 113 may be provided on another structure like the frame 112.

The mounting groove 113 is provided with a communication hole 114 that communicates the accommodation space A, and the communication hole 114 penetrates through the top surface 1111 and a groove wall of the mounting groove 113. The communication hole 114 is used to implement communication between the airbag 50 and parts of the blood pressure measurement component 14 located in the accommodation space A. In this implementation, there is one communication hole 114. Certainly, in another implementation, there may be one or more communication holes. Alternatively, in another implementation, the communication hole is not provided in the mounting groove, and the communication hole may be provided in another location of the bottom wall 111 or the housing 11.

As shown in FIG. 4 and FIG. 5. FIG. 5 is a schematic exploded diagram of a structure of the electronic device shown in FIG. 2.

In some implementations, the electronic device 100 further includes a fastener 15. The fastener 15 is fastened in the mounting groove 113, and is located on a side that is of the airbag 50 and that is away from the bottom wall 111, to fasten the airbag 50, thereby preventing the airbag 50 from falling off from the communication hole 114, and ensuring stability of communication between the airbag 50 and the blood pressure measurement component 14 through the communication hole 114. The fastener 15 may further prevent the airbag 50 from falling off from the mounting groove 113, thereby improving mounting stability between the airbag 50 and the bottom wall 111.

The display 12 includes a display panel and a cover fastened to the display panel. The cover may be made of transparent materials such as glass. The display panel may be an LCD (Liquid Crystal Display, liquid crystal display), an OLED (Organic Light-Emitting Diode, organic light-emitting diode) display, or an AMOLED (Active-Matrix Organic Light-Emitting Diode, active-matrix organic light-emitting diode) display, an FLED (Flex Light-Emitting Diode, flex light-emitting diode) display, a mini LED, a micro LED, a micro OLED, a QLED (Quantum Dot Light Emitting Diode, quantum dot light emitting diode), and the like.

The display panel may further integrate a touch control function. That is, the display panel is a touch control display panel. To be specific, the display panel may be used as not only an input apparatus for receiving input, but also an apparatus for providing output. In other words, the display 12 is a touchscreen. The display panel is electrically connected to the processor 13. The display panel can generate a touch control signal and transfer the touch control signal to the processor 13. The processor 13 receives the touch control signal, and controls enabling of an application (Application, App) in the watch body 10 based on the touch control signal. For example, the user may touch or press a location of a graph on the display 12 to choose to open or edit the graph.

The processor 13 is accommodated in the accommodation space A. The processor 13 may include one or more processing units. For example, the processor unit may include an application processor (Application Processor, AP), a modem processor, a graphics processing unit (Graphics Processing Unit, GPU), an image signal processor (Image Signal Processor, ISP), a controller, a memory, a video codec, a digital signal processor (Digital Signal Processor, DSP), a baseband processor, a neural-network processing unit (Neural-network Processing Unit, NPU), and/or the like.

Different processing units may be independent devices, or may be integrated into one or more processors 13. The processor 13 may be a nerve center and a command center of the electronic device 100. The processor 13 may generate an operation control signal based on an instruction operation code and a time sequence signal to control instruction reading and instruction executing. In addition, the processor 13 may further include a storage unit configured to store instructions and data. For example, the storage unit of the processor 13 is a cache. The memory may store instructions or data that has been recently used or cyclically used by the processor 13. If the processor 13 needs to use the instructions or the data again, the processor may directly invoke the instructions or the data from the memory, to avoid repeated access and reduce waiting time of the processor 13, thereby improving system efficiency.

As shown in FIG. 4, the blood pressure measurement component 14 is electrically connected to the processor 13. In this embodiment, the blood pressure measurement component 14 includes an air pump 141 and a pressure sensor 142. The air pump 141 is electrically connected to the processor 13, and is configured to receive a control signal sent by the processor 13 and extract or exhaust air based on the control signal. The pressure sensor 142 is spaced from the air pump 141, and is electrically connected to the processor 13. The pressure sensor 142 is configured to: receive the control signal sent by the processor 13, sense a pressure signal based on the control signal, and convert the pressure signal into an electrical signal. The pressure sensor 142 may be a capacitive pressure sensor. The capacitive pressure sensor includes at least two parallel plates made of conductive materials. When a force is applied to the pressure sensor 142, capacitance between electrodes changes. The processor 13 determines pressure intensity based on the change in the capacitance between electrodes. Certainly, the pressure sensor 142 may alternatively be a resistive pressure sensor, an inductive pressure sensor, or the like.

Specifically, both the air pump 141 and the pressure sensor 142 are located in the accommodation space A of the watch body 10. In addition, the air pump 141 communicates with the communication hole 114, to extract or exhaust air from the airbag 50 by using the communication hole 114. The pressure sensor 142 is disposed close to a joint between the communication hole 114 and the airbag 50, so that the pressure sensor 142 senses a change in atmospheric pressure to perform blood pressure measurement.

In some implementations, the watch body 10 further includes a sensing component. The sensing component is spaced from the blood pressure measurement component 14, and is electrically connected to the processor 13. Specifically, the bottom wall 111 includes a mounting hole that penetrates through the top surface 1111 and the bottom surface 1112. The sensing component includes a heart rate sensor, and the heart rate sensor directly faces the mounting hole (not shown in the figure) or is at least partially accommodated in the mounting hole. The heart rate sensor is an optical heart rate sensor. When the user wears the electronic device 100, the bottom surface 1112 of the bottom wall 111 is attached to the wrist of the user. The optical heart rate sensor may send a heart rate sensing signal through the mounting hole, to accurately detect a heart rate, thereby improving function diversity of the electronic device 100, and improving user experience. Certainly, the heart rate sensor may alternatively be a sensor that can detect the heart rate, for example, a bone conduction sensor.

In addition, the sensor component may further include sensors such as a gyroscope sensor, a barometric pressure sensor, an acceleration sensor, a distance sensor, an optical proximity sensor, a fingerprint sensor, a temperature sensor, or an ambient light sensor, to further improve function diversity of the electronic device 100 and improve user experience.

As shown in FIG. 3, in this embodiment, the watch body 10 may further include a Hall effect sensor 16. The Hall effect sensor 16 is disposed on the watch body 10 and is electrically connected to the processor 13. Specifically, the Hall effect sensor 16 may be disposed in the accommodation space A and disposed opposite to the mounting groove 113, or the Hall effect sensor 16 may be disposed in the mounting groove 113. The Hall effect sensor 16 is a device based on a Hall effect, can identify a magnetic field change, and may output a voltage based on a detected magnetic field status, to sense a type of the strap 20 disposed on the watch body 10.

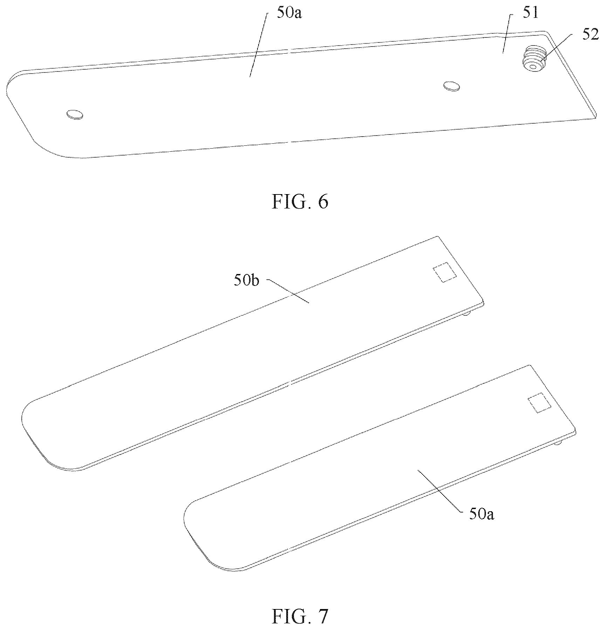

FIG. 6 is a schematic diagram of a structure of a first strap body of the electronic device shown in FIG. 5 from another angle.

It may be understood that there may be a plurality of types of straps 20, to adapt to different requirements of the user. For example, according to application requirements in different scenarios, when the user measures blood pressure, the strap 20 needs to have an airbag. When the user does not need to measure blood pressure, the strap 20 is more comfortable to wear without an airbag. Alternatively, according to application requirements of different users, blood pressure measurement is applicable to blood pressure measurement for a user, or may be applicable to blood pressure measurement for a family member of the user.

In this embodiment, there are two types of straps 20: a first strap and a second strap. A first airbag is disposed on the first strap, a second airbag is disposed on the second strap, and a length of the first airbag is different from a length of the second airbag. It may be understood that a length of the first strap is adapted to the length of the first airbag, and a length of the second strap is adapted to the length of the second airbag. It may be understood that either of the first strap and the second strap is disposed on the watch body 10. To be specific, one watch body matches one strap. The user may choose to dispose the first strap or the second strap on the watch body as required.

It may be understood that a prerequisite for obtaining an accurate blood pressure value is that the airbag of the strap 20 fully covers a radial artery and an ulnar artery. However, an existing strap is designed to be long to adapt to users with different wrist circumferences for wearing. For a user with a small wrist circumference, an excessively long strap is inevitably entangled in the bottom of the watch body, affecting user experience. In view of this, there are two types of straps 20 in this embodiment. Different types of straps 20 have different airbag lengths. Users with different wrist circumferences may select straps 20 with different airbag lengths as required. On the premise that it can be ensured that the airbag of the strap 20 can fully cover the radial artery and the ulnar artery, it can be further ensured that a length of the strap 20 is not excessively long, thereby ensuring user experience.

In this embodiment, how to fit the first airbag with the first strap is the same as how to fit the second airbag with the second strap. The following uses fitting the first airbag with the first strap as an example for description. The first airbag is disposed on an inner side of the first strap body 21, and is disposed in the mounting groove 113. Certainly, in another embodiment, the first airbag may alternatively be disposed on an inner side of the second strap body. Alternatively, the first airbag may be disposed on inner sides of the watch body and the first strap body. Alternatively, the first airbag may be disposed on inner sides of the first strap body, the watch body, and the second strap body.

In this implementation, a specific structure of the first airbag is the same as a specific structure of the second airbag, but lengths are different. The following uses the first airbag as an example for description. As shown in FIG. 5 and FIG. 6, a first airbag 50a includes a mounting part 51. When the first airbag 50a is disposed in the corresponding mounting groove 113, the mounting part 51 of the first airbag 50a is disposed in the mounting groove 113, and the fastener 15 is located on a surface that is of the mounting part 51 and that is opposite to the mounting groove 113, to dispose the first airbag 50a on the watch body 10. An air nozzle 52 is disposed on the mounting part 51. When the first airbag 50a is disposed in the corresponding mounting groove 113, the air nozzle 52 is inserted into the communication hole 114 of the housing, to implement communication between the first airbag 50a and the air pump 141, so that the air pump 141 extracts or exhausts air from the first airbag 50a.

Certainly, in another implementation, the first airbag and the second airbag may have a same length but different widths. Alternatively, the first airbag and the second airbag may have different lengths and widths. Alternatively, the first airbag and the second airbag may have different volumes. To be specific, the first airbag and the second airbag have different inflatable amounts.

Refer to FIG. 4 and FIG. 6 together. In some implementations, a groove is provided around a periphery of the air nozzle 52. A sealing ring 53 is disposed in the groove, to seal a gap between the air nozzle 52 and a hole wall of the communication hole 114, thereby ensuring that the first airbag 50a does not leak air. Certainly, in some other implementations, sealing between the air nozzle 52 and the communication hole 114 may be implemented by using other structures other than the sealing ring 53.

It may be understood that how to install the second airbag in the mounting groove 113 corresponding to the watch body 10 may be the same as or different from how to install the first airbag 50a, and details are not described herein again.

When the user wears the electronic device 100 on the wrist of the user, the first airbag 50a is attached to the wrist of the user. When the electronic device 100 receives a blood pressure measurement instruction, the processor 13 sends a blood pressure measurement signal to the air pump 141 and the pressure sensor 142. The air pump 141 performs inflating and pressurization on the first airbag 50a by using the communication hole 114 and the air nozzle 52. In this case, the first airbag 50a bulges and presses the radial artery and the ulnar artery of the user. The pressure sensor 142 detects an atmospheric pressure value in the first airbag 50a and feeds back the value to the processor 13 in real time. The processor 13 determines whether the atmospheric pressure value in the first airbag 50a meets a requirement for blood pressure measurement. If the atmospheric pressure value in the first airbag 50a does not meet the requirement for blood pressure measurement, the blood pressure measurement signal continues to be sent to the air pump 141, and the air pump 141 continues to perform inflating and pressurization on the first airbag 50a until the processor 13 determines that the atmospheric pressure value fed back by the pressure sensor 142 meets the requirement for blood pressure measurement. The processor 13 calculates blood pressure of the user based on the atmospheric pressure value fed back by the pressure sensor 142 in real time, and feeds back the blood pressure to the user by displaying the blood pressure on the display 12. After the blood pressure measurement is completed, the processor 13 stops sending the blood pressure measurement signal. Both the air pump 141 and the pressure sensor 142 stop working. Air in the first airbag 50a is transmitted to the air pump 141, and is exhausted by using the air pump 141. In this case, the first airbag 50a is restored to be flat and is attached to the wrist of the user.

For example, in this implementation, as shown in FIG. 7, a length of the first airbag 50a is less than a length of a second airbag 50b. Correspondingly, a length of a first strap body of the first strap is less than a length of a first strap body of the second strap. When the strap 20 is the first strap, the first strap is a short airbag strap. When the strap 20 is the second strap, the second strap is a long airbag strap. The first strap is applicable to a user with a small wrist circumference, and the second strap is applicable to a user with a large wrist circumference. For example, the first strap may be applicable to a user with a wrist circumference ranging from 130 mm to 160 mm (including 130 mm and 160 mm), and the second strap may be applicable to a user with a wrist circumference ranging from 161 mm to 230 mm (including 230 mm). It may be understood that lengths of second strap bodies 22 of the first strap and the second strap may be the same or different.

In this embodiment, as shown in FIG. 5, a magnet may be further disposed on the strap 20. Specifically, the magnet is disposed on the mounting part of the airbag 50. When the airbag 50 is disposed in the mounting groove 113, the magnet and the Hall effect sensor 16 are disposed opposite to each other. For example, it may be understood that a magnet disposed on the first strap is a first magnet 54, and a magnet disposed on the second strap is a second magnet. When the strap 20 is the first strap, a first magnetic pole of the first magnet 54 disposed on the first airbag 50a faces the Hall effect sensor 16. When the strap 20 is the second strap, a second magnetic pole of the second magnet disposed on the second airbag faces the Hall effect sensor 16. The first magnetic pole is different from the second magnetic pole. In this embodiment, the first magnetic pole is an S pole, and the second magnetic pole is an N pole. Certainly, in another embodiment, the first magnetic pole may alternatively be an N pole, and the second magnetic pole may alternatively be an S pole.

Certainly, in another embodiment, the first magnet may be further disposed on the mounting part of the first strap, and the second magnet may be disposed on the mounting part of the second strap.

When the first strap body 21 is disposed on the watch body 10, if the Hall effect sensor 16 detects a magnetic field of the first magnetic pole, the processor 13 determines that the first strap is disposed on the watch body 10. If the Hall effect sensor 16 detects a magnetic field of the second magnetic pole, the processor 13 determines that the second strap is disposed on the watch body 10. The processor 13 may send a detected result to the display 12 (FIG. 1), to present the detection result to the user. The user may learn, based on the detected result, whether the user wears an incorrect strap, thereby avoiding a problem that blood pressure precision decreases because the user uses the incorrect strap.

In this embodiment, the Hall effect sensor 16 is disposed on the watch body 10, and the magnet is disposed on the airbag 50. Magnetic poles that are of magnets disposed on airbags 50 of different types of straps 20 and that face the Hall effect sensor 16 are different, so that the Hall effect sensor 16 can obtain, by correspondingly identifying a detected type of a magnetic pole, a type of a strap 20 disposed on the watch body 10. The user can learn, based on an identification result, whether the user wears an incorrect strap, thereby avoiding a problem that blood pressure precision decreases because the user uses the incorrect strap.

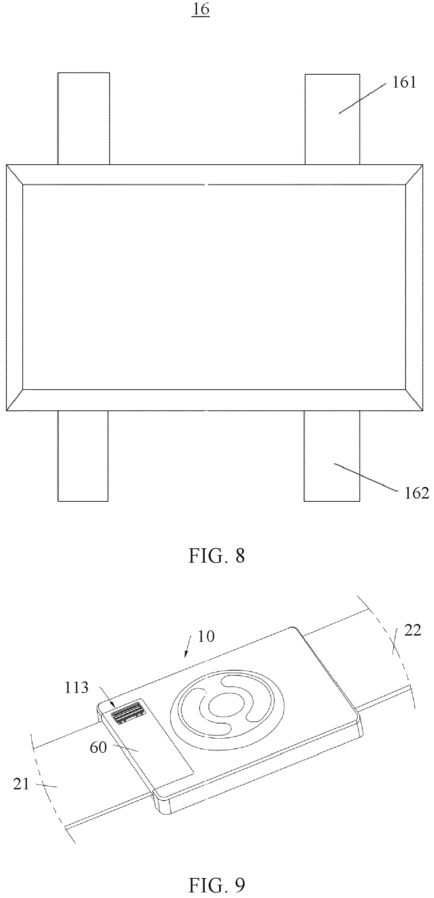

It may be understood that, as shown in FIG. 8, the Hall effect sensor 16 includes first output pins 161 and second output pins 162. The Hall effect sensor 16 can identify a change in a magnetic field, including an N-pole magnetic field, an S-pole magnetic field, and no magnetic field. When the strap 20 is disposed on the watch body 10, if the strap 20 is the first strap, and an S pole of the first airbag 50a faces the Hall effect sensor 16, it is detected that the first output pin 161 of the Hall effect sensor 16 has a low level, and the second output pin 162 has a high level. If the strap 20 is the second strap, and an N pole of the second airbag faces the Hall effect sensor 16, it is detected that the first output pin 161 of the Hall effect sensor 16 has a high level, and the second output pin 162 has a low level.

In this embodiment, the Hall effect sensor 16 can further identify a case in which there is no magnetic field. When the airbag 50 is not disposed on the watch body 10, levels of both the first output pin 161 and the second output pin 162 of the Hall effect sensor 16 are detected as low levels. It may be understood that, a case that the Hall effect sensor 16 detects that there is no magnetic field may alternatively be a case that the airbag 50 is loosely disposed. When receiving that the airbag 50 is not disposed on the watch body 10 of the electronic device 100, the user may dispose the airbag 50 on the watch body 10 or check whether the airbag 50 is loose, to avoid that the user washes hands or takes a shower to cause water damage to the device when the airbag 50 is loose.

Certainly, in some other embodiments, the electronic device 100 may alternatively identify the type of the strap 20 without using collaboration between the Hall effect sensor and the magnet. In other words, the electronic device 100 may alternatively not include the Hall effect sensor and the magnet. The processor 13 can further control the air pump 141 to inflate the strap 20, to obtain test data, and detect that the strap 20 is the first strap or the second strap based on the test data. Specifically, the air pump 141 in the watch body 10 may inflate the airbag 50. Because lengths of airbags of the first strap and the second strap are different, when the airbags of the first strap and the second strap are inflated, atmospheric pressure changes identified by the pressure sensor 142 are also different. Pressurization curve slopes of the first strap and the second strap in an inflated state are different, and the pressurization curve slope is the test data. Whether the strap 20 is the first strap or the second strap is detected by determining the pressurization curve slope of the strap 20 in the inflated state.

The following describes a second embodiment of an electronic device 100 in this application. A structure of the electronic device 100 in this embodiment is roughly the same as a structure of the electronic device 100 in the first embodiment. For parts of the same structure, refer to the accompanying drawings of the first embodiment. A difference lies in that there are three types of straps 20 in this embodiment: a short airbag strap, a long airbag strap, and a common strap. Airbags are disposed on both the short airbag strap and the long airbag strap, and an airbag is not disposed on the common strap. A first airbag is disposed on the short airbag strap, and a second airbag is disposed on the long airbag strap. A length of the first airbag is different from a length of the second airbag, and the common strap includes a cover. The short airbag strap is a first strap, and the long airbag strap and the common strap are second straps. Certainly, in another embodiment, the long airbag strap may be a first strap, and the short airbag strap and the common strap may be second straps.

It may be understood that a prerequisite for obtaining an accurate blood pressure value is that the airbag of the strap 20 fully covers a radial artery and an ulnar artery. However, an existing strap is designed to be long to adapt to users with different wrist circumferences for wearing. For a user with a small wrist circumference, an excessively long strap is inevitably entangled in the bottom of the watch body, affecting user experience. In addition, the electronic device not only has a function attribute of measuring blood, but also has another measurement function or time display function. When the electronic device is used without any need for blood pressure measurement, wearing comfort of a strap with an airbag is poor.

In view of this, there are three types of straps 20 in this embodiment, including the long airbag strap, the short airbag strap, and the common strap. During blood pressure measurement, users with different wrist circumferences may select straps 20 with different airbag lengths as required. On the premise that it can be ensured that the airbag of the strap 20 can fully cover the radial artery and the ulnar artery, it can be further ensured that a length of the strap 20 is not excessively long, thereby ensuring user experience. When there is no need for blood pressure measurement, the user may select the common strap, to improve wearing comfort.

In this embodiment, for how to fit the first airbag with the short airbag strap and how to fit the second airbag with the long airbag strap, refer to how to fit the first airbag with the first strap in the first embodiment. Details are not described again. It may be understood that, because the first airbag may be detachably connected to a first strap body of the short airbag strap and a watch body, the second airbag may be detachably connected to a first strap body of the long airbag strap and a watch body. The following uses the short airbag strap as an example for description.

As shown in FIG. 9, after the first airbag is detached from the first strap body of the short airbag strap and from a mounting groove of the watch body, a cover 60 covers the mounting groove 113. The cover 60 includes an air nozzle, and the air nozzle is disposed in a vent hole of the mounting groove 113, to seal the vent hole, so that the short airbag strap becomes the common strap. Similarly, the long airbag strap may also become the common strap by detaching the second airbag. Certainly, in another embodiment, after the first airbag is directly detached from the short airbag strap, the short airbag strap may also be the common strap.

A length of the first airbag is less than a length of the second airbag. Correspondingly, a length of the first strap body of the short airbag strap is less than a length of the first strap body of the long airbag strap. The short airbag strap is applicable to a user with a small wrist circumference, and the long airbag strap is applicable to a user with a large wrist circumference. When the strap 20 is the common strap, the cover 60 covers the mounting groove. For example, the short airbag strap may be applicable to a user with a wrist circumference ranging from 130 mm to 160 mm (including 130 mm and 160 mm), and the long airbag strap may be applicable to a user with a wrist circumference ranging from 161 mm to 230 mm (including 230 mm). It may be understood that lengths of second strap bodies 22 of the short airbag strap and the long airbag strap may be the same or different. The common strap is applicable to a scenario in which a user does not measure blood pressure.

In this embodiment, a magnet may be further disposed on the strap 20. Specifically, the magnet is disposed on a mounting part 51 of the airbag. When the airbag is disposed in the mounting groove 113, the magnet and a Hall effect sensor 16 are disposed opposite to each other. It may be understood that a magnet disposed on the first strap is a first magnet 54, and a magnet disposed on the second strap is a second magnet. For example, when the strap 20 is the short airbag strap, a first magnetic pole of the first magnet 54 disposed on a first airbag 50a faces the Hall effect sensor 16. When the strap 20 is the second strap (the long airbag strap or common strap), a second magnetic pole of the second magnet disposed on the second airbag or a second magnetic pole disposed on the cover faces the Hall effect sensor 16. The first magnetic pole is different from the second magnetic pole. In this embodiment, the first magnetic pole is an S pole, and the second magnetic pole is an N pole. Certainly, in another embodiment, the first magnetic pole may alternatively be an N pole, and the second magnetic pole may alternatively be an S pole.

Certainly, in another embodiment, the first magnet may be further disposed on the mounting part of the first strap, and when the strap is the long airbag strap, the second magnet may be disposed on the mounting part of the second strap.

When the first strap body 21 is disposed on the watch body 10, if the Hall effect sensor 16 detects a magnetic field of the first magnetic pole, a processor 13 determines that the strap 20 is the short airbag strap. If the Hall effect sensor 16 detects a magnetic field of the second magnetic pole, the processor 13 determines that the strap 20 is the second strap. Then, the processor 13 drives an air pump to inflate the second strap, to obtain test data. Then the test data is compared with preset data, to determine a type of the test data. If the test data is first data, the second strap is the long airbag strap. If the test data is second data, the second strap is the common strap. The processor 13 may send a detected result to a display 12, to present the detected result to the user. The user may learn, based on the detected result, whether the user wears an incorrect strap, thereby avoiding a problem that blood pressure precision decreases because the user uses the incorrect strap.

It may be understood that the air pump 141 in the watch body 10 may inflate the second strap. Because the long airbag strap has an airbag, and the common strap does not have an airbag, a pressure sensor 142 identifies different atmospheric pressure changes when the long airbag strap and the common strap are in an inflated state. Because the common strap does not have the airbag and cannot store air, a pressurization curve slope of the common strap increases rapidly in a short time according to a fixed slope. The long airbag strap has the airbag, and a pressurization curve slope of the long airbag strap increases slowly in a period of time according to a fixed slope.

Pressurization curve slopes of the long airbag strap and the common strap in the inflated state are different. The pressurization curve slope is the test data. A pressurization curve slope of the second strap is compared with preset data (the pressurization curve slope of the long airbag strap and the pressurization curve slope of the common strap). If the pressurization curve slope of the second strap matches the pressurization curve slope of the long airbag strap, it indicates that the test data is the first data, and the second strap is the long airbag strap. If the pressurization curve slope of the second strap matches the pressurization curve slope of the common strap, it indicates that the test data is the second data, and the second strap is the common strap.

In this embodiment, the Hall effect sensor 16 is disposed on the watch body 10, and the magnet is disposed on the airbag or the cover. Magnetic poles that are of magnets disposed on airbags or covers of the first strap and the second strap and that face the Hall effect sensor 16 are different, so that the Hall effect sensor 16 can obtain, by correspondingly identifying a detected type of a magnetic pole, whether the strap 20 disposed on the watch body 10 is the first strap or the second strap. If the strap 20 is the first strap, it may be learned that the strap 20 is the short airbag strap. If the strap 20 is the second strap, the second strap is further inflated to identify whether the second strap is the long airbag strap or the common strap. The processor 13 presents an identification result to the user by using a display 12, and the user may learn, based on the identification result, whether the user wears an incorrect strap, thereby avoiding a problem that blood pressure precision decreases because the user uses the incorrect strap.

In this embodiment, a case that the Hall effect sensor 16 detects that there is no magnetic field may alternatively be a case that the airbag 50 or the cover is loosely disposed. When receiving that the airbag 50 or the cover is not disposed on the watch body 10 of the electronic device 100, the user may dispose the airbag 50 or the cover on the watch body 10 or check whether the airbag 50 or the cover is loose, to avoid that the user washes hands or takes a shower to cause water damage to the device when the airbag 50 is loose.

Certainly, in another embodiment, when the Hall effect sensor 16 identifies a magnetic pole of the strap 20, the air pump 141 in the watch body 10 may inflate the strap 20, to determine, in a short time, whether the strap 20 is the common strap. Therefore, the processor 13 may quickly determine a type of the strap 20 in the short time based on a joint detection result of how the Hall effect sensor 16 and the magnetic pole collaborate and how to inflate the strap 20.

Certainly, in another embodiments, the electronic device 100 may alternatively directly identify, by inflating the strap 20, that the strap 20 is the short airbag strap, the long airbag strap, or the common strap. In this identification manner, the Hall effect sensor 16 and the magnet may not be disposed on the electronic device 100, so that components of the electronic device 100 can be reduced, costs can be reduced, and integration of the electronic device 100 can be improved. However, identification time of identifying the strap 20 by inflating the strap 20 is longer compared with identification time of first identifying the first strap and the second strap by collaboration between the Hall effect sensor 16 and the magnet, and then further identifying the long airbag strap and the common strap by inflating the strap 20, and user experience is not as good as user experience of the latter.

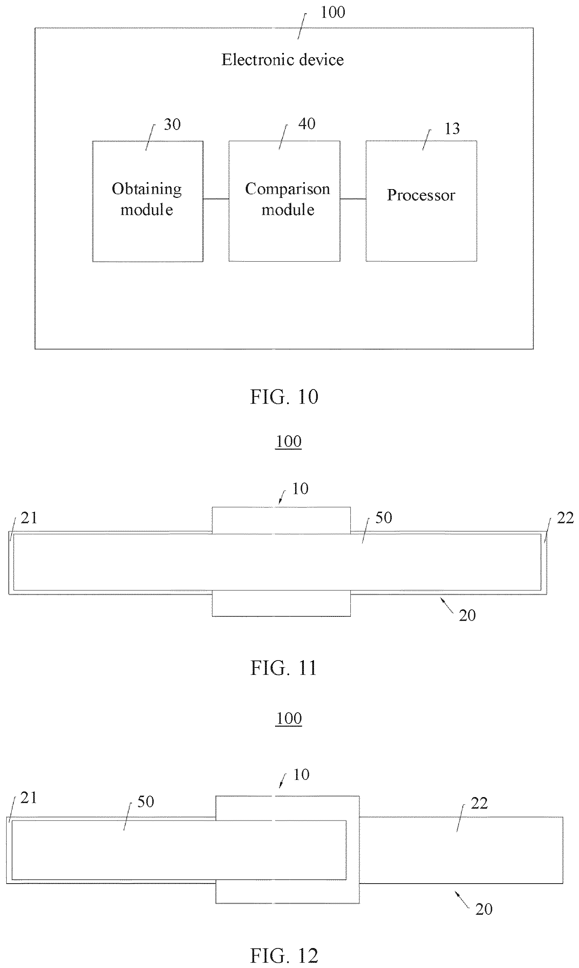

The following describes a third embodiment of an electronic device 100 in this application. A structure of the electronic device 100 in this embodiment is roughly the same as a structure of the electronic device 100 in the second embodiment. For parts of the same structure, refer to the accompanying drawings of the second embodiment. A difference lies in that, as shown in FIG. 10, the electronic device 100 further includes an obtaining module 30 and a comparison module 40, and both the obtaining module 30 and the comparison module 40 are electrically connected to a processor 13. Certainly, in some implementation, the obtaining module 30 and the comparison module 40 may also be a part of the processor 13.

The obtaining module 30 is configured to obtain wrist circumference data of a user. For example, a watch body may include a button. A display or the button of the electronic device is configured to receive an input operation of the user. The obtaining module 30 is configured to obtain an electrical signal of the display or the button, to obtain the wrist circumference data input by the user. The comparison module 40 is configured to compare the wrist circumference data with a type of a strap 20, to detect that the strap 20 is selected correctly or incorrectly. If the strap 20 is incorrectly selected, the processor 13 reminds the user to replace the strap 20 with a correct strap 20. If the strap 20 is correctly selected, blood pressure measurement is performed. Certainly, in another embodiment, the obtaining module 30 may further obtain the wrist circumference data of the user through automatic measurement.

In this implementation, the type of the strap 20 is identified, and the type of the strap 20 is compared with the wrist circumference data of the user, to determine that the user selects a strap 20 for installation correctly or incorrectly. If the strap 20 is correctly selected, blood pressure measurement is performed. If the strap 20 is incorrectly selected, the user is reminded to replace the strap 20 with a correct strap 20, so that the user is prevented from selecting an incorrect strap 20, and blood pressure measurement is more accurate.

In some other embodiments, FIG. 11 is a schematic diagram of a structure of the electronic device shown in FIG. 2 according to another embodiment.

The structure of this embodiment is roughly the same as the structure of the embodiment shown in FIG. 2, and the same part is not described again. A difference lies in that there are three types of straps 20 in this embodiment: a short airbag strap, a long airbag strap, and a common strap. Airbags are disposed on both the short airbag strap and the long airbag strap, and an airbag is not disposed on the common strap. A first airbag is disposed on the short airbag strap, and a second airbag is disposed on the long airbag strap. A length of the first airbag is different from a length of the second airbag, and the common strap includes a cover. The short airbag strap is a first strap, and the long airbag strap and the common strap are second straps.

In this embodiment, an airbag 50 is disposed on inner sides of a first strap body, a watch body, and a second strap body. When the strap 20 is the short airbag strap, the first airbag is disposed on inner sides of the first strap body, the watch body, and the second strap body. When the strap 20 is the long airbag strap, the second airbag is disposed on inner sides of the first strap body, the watch body, and the second strap body. When the strap 20 is the common strap, the cover is disposed on the watch body.

A length of the first airbag is less than a length of the second airbag, and a length of the short airbag strap is less than a length of the long airbag strap. The short airbag strap is applicable to a user with a small wrist circumference, and the long airbag strap is applicable to a user with a large wrist circumference. For example, the short airbag strap may be applicable to a user with a wrist circumference ranging from 130 mm to 160 mm (including 130 mm and 160 mm), and the long airbag strap may be applicable to a user with a wrist circumference ranging from 161 mm to 230 mm (including 161 mm and 230 mm). The common airbag is applicable to a scenario without blood pressure measurement.

In this embodiment, an air nozzle of the airbag 50 may be disposed at a part that is of the airbag and that is close to the watch body. This is not limited in this application.

In some other embodiments, FIG. 12 is a schematic diagram of a structure of the electronic device shown in FIG. 2 according to another embodiment.

The structure of this embodiment is roughly the same as the structure of the embodiment shown in FIG. 11, and the same part is not described again. A difference lies in that there are three types of straps 20 in this embodiment: a short airbag strap, a long airbag strap, and a common strap. Airbags are disposed on both the short airbag strap and the long airbag strap, and an airbag is not disposed on the common strap. A first airbag is disposed on the short airbag strap, and a second airbag is disposed on the long airbag strap. A length of the first airbag is different from a length of the second airbag, and the common strap includes a cover. The short airbag strap is a first strap, and the long airbag strap and the common strap are second straps. In this embodiment, an airbag 50 is disposed on inner sides of a first strap body and a watch body. When the strap 20 is the short airbag strap, the first airbag is disposed on inner sides of the first strap body and the watch body. When the strap 20 is the long airbag strap, the second airbag is disposed on inner sides of the first strap body and the watch body. When the strap 20 is the common strap, the cover is disposed on the watch body.

A length of the first airbag is less than a length of the second airbag, and a length of the short airbag strap is less than a length of the long airbag strap. The short airbag strap is applicable to a user with a small wrist circumference, and the long airbag strap is applicable to a user with a large wrist circumference. For example, the short airbag strap may be applicable to a user with a wrist circumference ranging from 130 mm to 160 mm (including 130 mm and 160 mm), and the long airbag strap may be applicable to a user with a wrist circumference ranging from 161 mm to 230 mm (including 161 mm and 230 mm). The common airbag is applicable to a scenario without blood pressure measurement.

In this embodiment, an air nozzle of the airbag 50 may be disposed at a part that is of the airbag and that is close to the watch body. This is not limited in this application.

The foregoing specifically describes a structure of the electronic device 100. The following describes a strap 20 identification method of the electronic device 100 with reference to the foregoing structure of the electronic device 100 in the first embodiment.

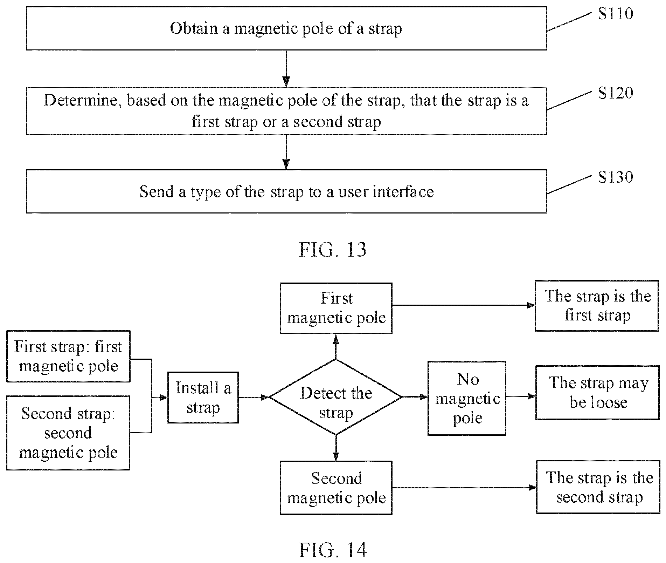

FIG. 13 is a schematic flowchart of a strap 20 identification method of the electronic device 100 according to a first embodiment. The strap 20 identification method of the electronic device 100 includes the following steps S110 to S130.

S110: Obtain a magnetic pole of a strap 20.

Specifically, as shown in FIG. 5 and FIG. 14, the electronic device 100 includes a watch body 10 and the strap 20. An airbag 50 is disposed on an inner side of the strap 20. In this embodiment, there are two types of straps 20: a first strap and a second strap. A first airbag is disposed on the first strap, and a second airbag is disposed on the second strap. A length of the first airbag is different from a length of the second airbag. Therefore, a user may select a suitable strap 20 based on a wrist circumference of the user.

A Hall effect sensor 16 is disposed on the watch body 10, and a magnet is disposed on the airbag 50 of the strap 20. Magnets are disposed on the airbags of the first strap and the second strap in different manners. When the strap 20 is the first strap, the strap 20 is disposed on the watch body 10, and a first magnetic pole of a first magnet is disposed opposite to the Hall effect sensor 16 of the watch body 10. When the strap 20 is the second strap, the strap 20 is disposed on the watch body 10, and a second magnetic pole of a second magnet is disposed opposite to the Hall effect sensor 16 of the watch body 10. The first magnetic pole is different from the second magnetic pole. The first magnetic pole is an S pole, and the second magnetic pole is an N pole. Because the Hall effect sensor 16 can detect a magnetic field change, and magnetic poles of different straps 20 are different, the watch body 10 can identify a magnetic pole to obtain whether the strap 20 is the first strap or the second strap.

Before the magnetic pole of the strap 20 is obtained, the method further includes: receiving a strap 20 identification instruction. It may be understood that when a processor 13 detects some operations of the user, the processor 13 may send the strap 20 identification instruction to the Hall effect sensor 16. For example, when the processor 13 detects that the user starts the electronic device 100 for the first time, when the processor 13 detects that the user opens a blood pressure measurement function page of the electronic device 100, and when the processor 13 detects that the user disposes the strap 20 on the watch body 10. After receiving the strap 20 identification instruction, the Hall effect sensor 16 detects the magnetic pole of the strap 20, to obtain the magnetic pole of the strap 20.

S120: Determine, based on the magnetic pole of the strap 20, that the strap 20 is the first strap or the second strap.

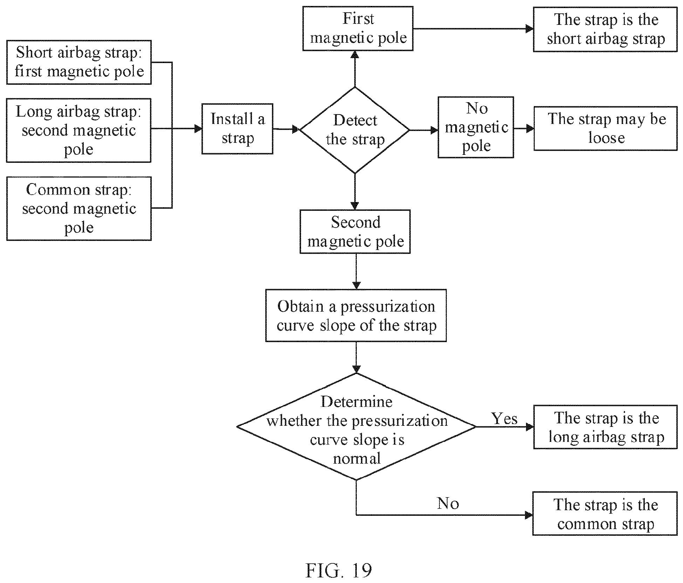

Specifically, as shown in FIG. 19, if the Hall effect sensor detects a magnetic field of the first magnetic pole, the strap 20 is the first strap. If the Hall effect sensor detects a magnetic field of the second magnetic pole, the strap 20 is the second strap. The first strap is a short airbag strap, and the second strap is a long airbag strap. The first strap is applicable to a user with a small wrist circumference, and the second strap is applicable to a user with a large wrist circumference. For example, the first strap may be applicable to a user with a wrist circumference ranging from 130 mm to 160 mm (including 130 mm and 160 mm), and the second strap may be applicable to a user with a wrist circumference ranging from 161 mm to 230 mm (including 161 mm and 230 mm).

Certainly, if the magnetic pole of the strap 20 is no magnetic pole, the airbag is not disposed on the watch body 10, or the airbag is loose from the watch body 10.

S130: Send a type of the strap 20 to a user interface.

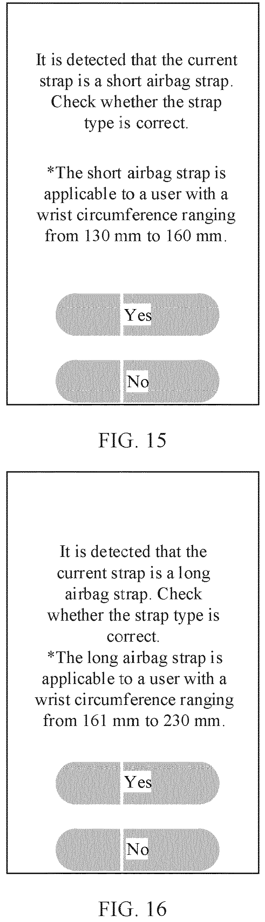

Specifically, when it is detected in step S120 that the strap 20 is the first strap, as shown in FIG. 15, "It is detected that the current strap is a short airbag strap. Check whether the strap type is correct" is sent to the user interface. If the user confirms that the type of the strap 20 is correct, the user taps "Yes". If the user confirms that the type of the strap 20 is incorrect, the user taps "No". In some embodiments, when "It is detected that the current strap is a short airbag strap. Check whether the strap type is correct" is displayed, "The short airbag strap is applicable to a user with a wrist circumference ranging from 130 mm to 160 mm" is further be noted below "It is detected that the current strap is a short airbag strap. Check whether the strap type is correct".

When it is detected in step S120 that the strap 20 is the second strap, as shown in FIG. 16, "It is detected that the current strap is a long airbag strap. Check whether the strap type is correct" is sent to the user interface. If the user confirms that the type of the strap 20 is correct, the user taps "Yes". If the user confirms that the type of the strap 20 is incorrect, the user taps "No". In some embodiments, when "It is detected that the current strap is a long airbag strap. Check whether the strap type is correct" is displayed, "The long airbag strap is applicable to a user with a wrist circumference ranging from 161 mm to 230 mm" is further noted below "It is detected that the current strap is a long airbag strap. Check whether the strap type is correct".

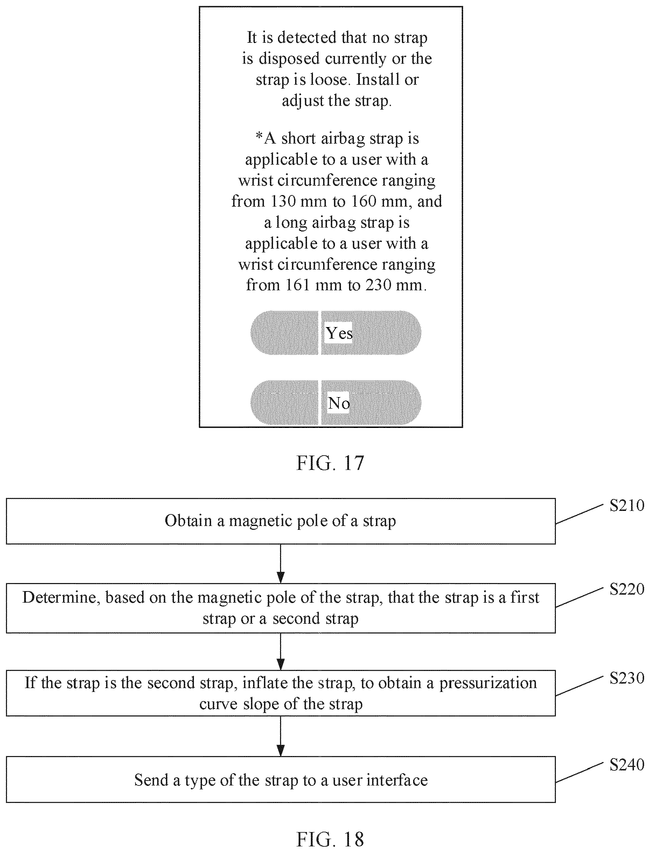

When it is detected in step S120 that the strap 20 is not disposed on the watch body 10 or the strap 20 is loose, as shown in FIG. 17, "It is detected that no strap is disposed currently or the strap is loose. Install or adjust the strap" is sent to the user interface. In some implementations, "A short airbag strap is applicable to a user with a wrist circumference ranging from 130 mm to 160 mm, and a long airbag strap is applicable to a user with a wrist circumference ranging from 161 mm to 230 mm. Select a proper strap for installation" is further noted below the foregoing "It is detected that no strap is disposed currently or the strap is loose. Install or adjust the strap".

In this embodiment, the type of the strap 20 disposed on the watch body 10 is identified, and an identification result is sent to the user interface. The user may learn, based on the identification result, whether the user wears an incorrect strap, thereby avoiding a problem that blood pressure precision decreases because the user uses the incorrect strap. In addition, if the user receives that the strap 20 is not disposed on the watch body 10 of the electronic device 100, the user may dispose the strap 20 on the watch body 10 or check whether the strap 20 is loose, to avoid that the user washes hands or takes a shower to cause water damage to the device when the strap 20 is loose.

Certainly, in another embodiment, the electronic device 100 may alternatively identify the type of the strap 20 without using collaboration between the Hall effect sensor 16 and the magnet. For example, the electronic device 100 may identify the type of the strap 20 by inflating the strap 20. Specifically, after receiving the strap 20 identification instruction, the processor 13 controls an air pump 141 to inflate the strap 20, and a pressure sensor 142 obtains an atmospheric pressure change value, to obtain a pressurization curve slope of the strap 20 in an inflated state. It may be understood that airbag lengths of the long airbag strap and the short airbag strap are different, and therefore, pressurization curve slopes of the long airbag strap and the short airbag strap are different. The pressurization curve slopes of the long airbag strap and the short airbag strap may be stored in the processor 13 in advance. The obtained pressurization curve slope is compared with a pre-stored pressurization curve slope, to determine whether the strap 20 is the long airbag strap or the short airbag strap.

FIG. 18 is a schematic flowchart of a strap 20 identification method of the electronic device 100 according to a second embodiment. The strap 20 identification method of the electronic device 100 includes the following steps S210 to S240.

S210: Obtain a magnetic pole of a strap 20.

Specifically, refer to FIG. 5 and FIG. 19. The electronic device 100 includes a watch body 10 and the strap 20. An airbag 50 is disposed on an inner side of the strap 20. In this embodiment, there are three types of straps 20: a short airbag strap, a long airbag strap, and a common strap. Airbags are disposed on both the short airbag strap and the long airbag strap, and an airbag is not disposed on the common strap. A first airbag is disposed on the short airbag strap, and a second airbag is disposed on the long airbag strap. A length of the first airbag is different from a length of the second airbag, and the common strap includes a cover. The short airbag strap is a first strap, and the long airbag strap and the common strap are second straps.

During blood pressure measurement, users with different wrist circumferences may select straps 20 with different airbag lengths as required. On the premise that an airbag of the strap 20 can fully cover a radial artery and an ulnar artery, it can be further ensured that a length of the strap 20 is not excessively long, thereby ensuring user experience. When there is no need for blood pressure measurement, the user may select the common strap, to improve wearing comfort.

A Hall effect sensor 16 is disposed on the watch body 10, and a magnet is disposed on the airbag 50 or the cover of the strap 20. Magnets are disposed on the airbags or covers of the first strap and the second strap in different manners. When the strap 20 is the first strap, the strap 20 is disposed on the watch body 10, and a first magnetic pole of a first magnet is disposed opposite to the Hall effect sensor 16 of the watch body 10. When the strap 20 is the second strap, the strap 20 is disposed on the watch body 10, and a second magnetic pole of a second magnet is disposed opposite to the Hall effect sensor 16 of the watch body 10. The first magnetic pole is different from the second magnetic pole. The first magnetic pole is an S pole, and the second magnetic pole is an N pole. Because the Hall effect sensor 16 can detect a magnetic field change, and magnetic poles of different straps 20 are different, the watch body 10 can identify a magnetic pole to obtain whether the strap 20 is the first strap or the second strap.

Before the magnetic pole of the strap 20 is obtained, the method further includes: receiving a strap 20 identification instruction. It may be understood that when a processor 13 detects some operations of the user, the processor 13 may send the strap 20 identification instruction to the Hall effect sensor 16. For example, when the processor 13 detects that the user starts the electronic device 100 for the first time, when the processor 13 detects that the user opens a blood pressure measurement function page of the electronic device 100, and when the processor 13 detects that the user disposes the strap 20 on the watch body 10. After receiving the strap 20 identification instruction, the Hall effect sensor 16 detects the magnetic pole of the strap 20, to obtain the magnetic pole of the strap 20.

S220: Determine, based on the magnetic pole of the strap 20, that the strap 20 is the first strap or the second strap.

Specifically, as shown in FIG. 19, if the Hall effect sensor detects a magnetic field of the first magnetic pole, the strap 20 is the first strap, to be specific, the strap 20 is the short airbag strap. If the Hall effect sensor detects a magnetic field of the second magnetic pole, the strap 20 is the second strap. The first strap is applicable to a user with a small wrist circumference for blood pressure measurement. For example, the first strap may be applicable to a user with a wrist circumference ranging from 130 mm to 160 mm (including 130 mm and 160 mm).

Certainly, if the magnetic pole of the strap 20 is no magnetic pole, the airbag or the cover is not disposed on the watch body 10, or the airbag or the cover is loose from the watch body 10.

S230: If the strap 20 is the second strap, inflate the strap 20, to obtain a pressurization curve slope of the strap 20.

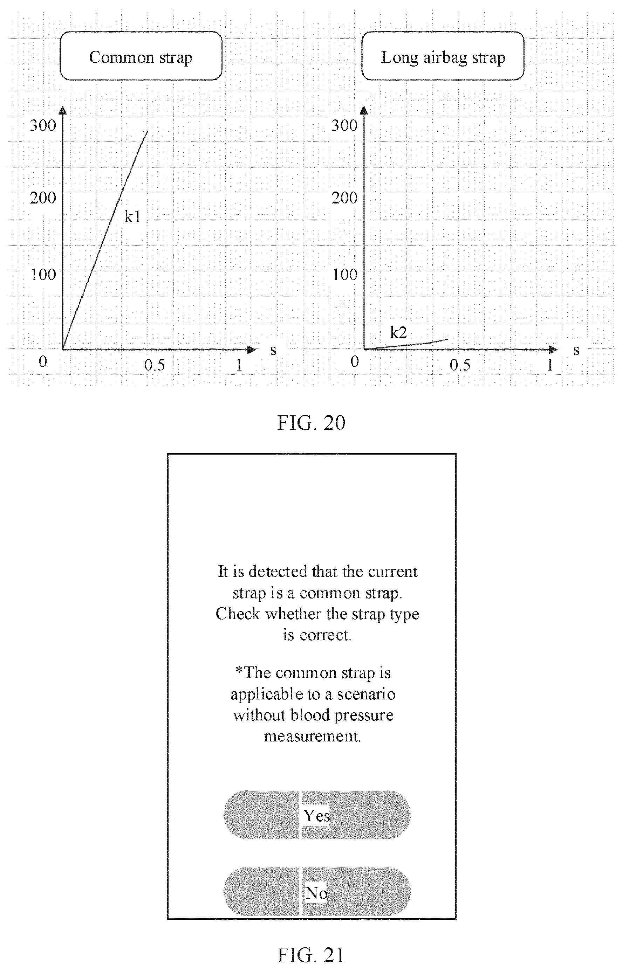

Specifically, as shown in FIG. 19, if the strap 20 is the second strap, it indicates that the second strap is the long airbag strap or the common strap. It may be understood that the air pump 141 in the watch body 10 may inflate the second strap. Because the long airbag strap has an airbag, and the common strap does not have an airbag, a pressure sensor 142 identifies different atmospheric pressure changes when the long airbag strap and the common strap are in an inflated state. As shown in FIG. 20, because the common strap does not have the airbag and cannot store air, a pressurization curve slope of the common strap increases rapidly in a short time according to a fixed slope (k1). The long airbag strap has the airbag, and a pressurization curve slope of the long airbag strap increases slowly in a period of time according to a fixed slope (k2).

Pressurization curve slopes of the long airbag strap and the common strap in the inflated state are different. The pressurization curve slope is test data. Test data of the second strap is compared with preset data (the pressurization curve slope of the long airbag strap and the pressurization curve slope of the common strap). If the pressurization curve slope of the second strap matches the pressurization curve slope of the long airbag strap, it indicates that the test data is first data, and the second strap is the long airbag strap. The second strap is applicable to a user with a large wrist circumference. For example, the second strap may be applicable to a user with a wrist circumference ranging from 161 mm to 230 mm (including 161 mm and 230 mm). If the pressurization curve slope of the second strap matches the pressurization curve slope of the common strap, it indicates that the test data is second data, and the second strap is the common strap. The common strap is applicable to wearing when the user does not measure blood pressure, thereby improving user comfort.

Certainly, in another embodiment, when the Hall effect sensor 16 identifies the magnetic pole of the strap 20, the air pump 141 in the watch body 10 may inflate the strap 20. In other words, step 220 and step 230 may be performed simultaneously, to determine, in a short time, whether the strap 20 is the common strap. Therefore, the processor 13 may quickly determine a type of the strap 20 in a short time based on a joint detection result of how the Hall effect sensor 16 and the magnetic pole collaborate and how to inflate the strap 20.

S240: Send the type of the strap 20 to a user interface.

Specifically, when it is detected in step S220 that the strap 20 is the first strap, as shown in FIG. 15, "It is detected that the current strap is a short airbag strap. Check whether the strap type is correct" is sent to the user interface. If the user confirms that the type of the strap 20 is correct, the user taps "Yes". If the user confirms that the type of the strap 20 is incorrect, the user taps "No". In some embodiments, when "It is detected that the current strap is a short airbag strap. Check whether the strap type is correct" is displayed, "The short airbag strap is applicable to a user with a wrist circumference ranging from 130 mm to 160 mm" is further be noted below "It is detected that the current strap is a short airbag strap. Check whether the strap type is correct".

When it is detected in step S230 that the strap 20 is the long airbag strap, as shown in FIG. 16, "It is detected that the current strap is a long airbag strap. Check whether the strap type is correct" is sent to the user interface. If the user confirms that the type of the strap 20 is correct, the user taps "Yes". If the user confirms that the type of the strap 20 is incorrect, the user taps "No". In some embodiments, when "It is detected that the current strap is a long airbag strap. Check whether the strap type is correct" is displayed, "The long airbag strap is applicable to a user with a wrist circumference ranging from 161 mm to 230 mm" is further noted below "It is detected that the current strap is a long airbag strap. Check whether the strap type is correct".

When it is detected in step S230 that the strap 20 is the common strap, as shown in FIG. 21, "It is detected that the current strap is a common strap. Check whether the strap type is correct" is sent to the user interface. If the user confirms that the type of the strap 20 is correct, the user taps "Yes". If the user confirms that the type of the strap 20 is incorrect, the user taps "No". In some embodiments, when "It is detected that the current strap is a common strap. Check whether the strap type is correct" is displayed, "The common strap is applicable to a scenario without blood pressure measurement" is further noted below "It is detected that the current strap is a common strap. Check whether the strap type is correct".

When it is detected in step S230 that the strap 20 is not disposed on the watch body 10 or the strap 20 is loose, "It is detected that no strap is disposed currently or the strap is loose. Install or adjust the strap" is sent to the user interface. In some implementations, "A short airbag strap is applicable to a user with a wrist circumference ranging from 130 mm to 160 mm, a long airbag strap is applicable to a user with a wrist circumference ranging from 161 mm to 230 mm, and a common strap is applicable to a scenario without blood pressure measurement. Select a proper strap for installation" is further noted below the foregoing "It is detected that no strap is disposed currently or the strap is loose. Install or adjust the strap".

In this embodiment, the type of the strap 20 disposed on the watch body 10 is identified, and an identification result is sent to the user interface. The user may learn, based on the identification result, whether the user wears an incorrect strap, thereby avoiding a problem that blood pressure precision decreases because the user uses the incorrect strap. In addition, if the user receives that the strap 20 is not disposed on the watch body 10 of the electronic device 100, the user may dispose the strap 20 on the watch body 10 or check whether the strap 20 is loose, to avoid that the user washes hands or takes a shower to cause water damage to the device when the strap 20 is loose.

Certainly, in another embodiment, the electronic device 100 may alternatively identify the type of the strap 20 without using collaboration between the Hall effect sensor 16 and the magnet 54. For example, the electronic device 100 may identify the type of the strap 20 by inflating the strap 20. Specifically, after receiving the strap 20 identification instruction, the processor 13 controls an air pump 141 to inflate the strap 20, and a pressure sensor 142 obtains an atmospheric pressure change value, to obtain a pressurization curve slope of the strap 20 in an inflated state. It may be understood that, airbag lengths of the long airbag strap and the short airbag strap are different, and the common strap does not have the airbag. Therefore, pressurization curve slopes of the long airbag strap, the short airbag strap, and the common strap are different, and the pressurization curve slopes of the long airbag strap, the short airbag strap, and the common strap may be stored in the processor 13 in advance. The obtained pressurization curve slope is compared with a pre-stored pressurization curve slope, to determine whether the strap 20 is the long airbag strap, the short airbag strap, or the common strap.

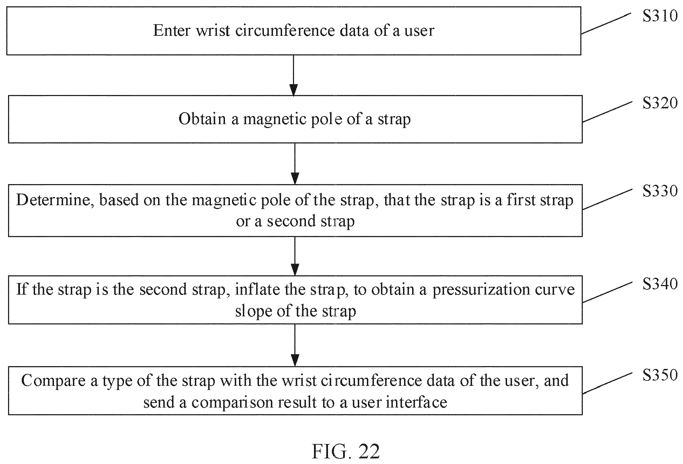

FIG. 22 is a schematic flowchart of a strap 20 identification method of the electronic device 100 according to a third embodiment. The strap 20 identification method of the electronic device 100 includes the following steps S310 to S350.

S310: Enter wrist circumference data of a user.

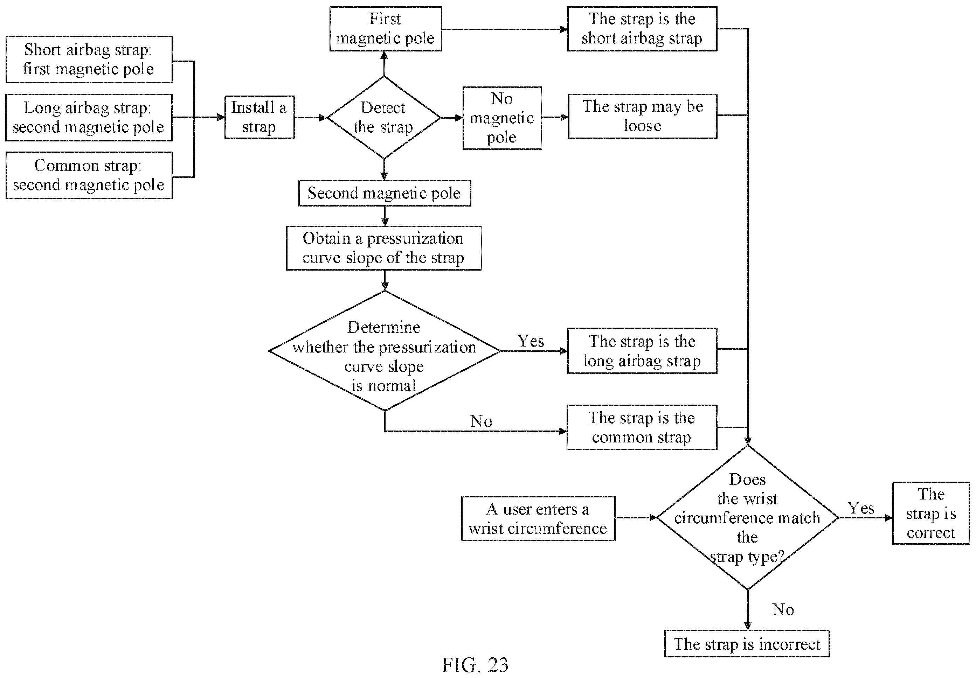

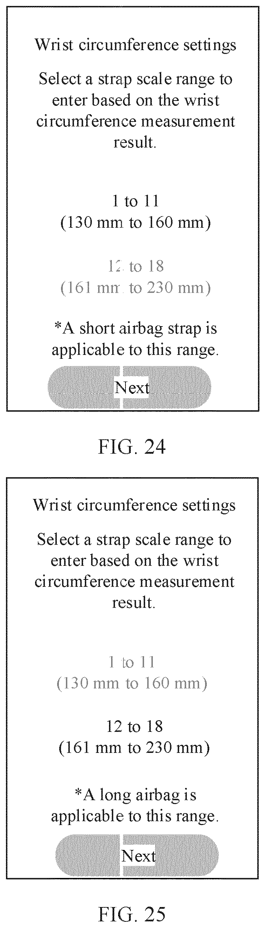

Specifically, as shown in FIG. 23, the wrist circumference data of the user may be entered into the electronic device 100 when the electronic device 100 is powered on. Specifically, when the electronic device 100 is used for the first time, the user may be guided for setting. For example, as shown in FIG. 24, a wrist circumference settings interface may pop up, and the interface prompts the user to "Select a strap scale range to enter based on the wrist circumference measurement result ". The user may select "1 to 11 (130 mm to 160 mm)" or "12 to 18 (161 mm to 230 mm)" based on the wrist circumference of the user, and may tap "Next". As shown in FIG. 24, when the user taps "1 to 11 (130 mm to 160 mm)", "A short airbag strap is applicable to this range" is displayed at the bottom of the interface. As shown in FIG. 25, when the user taps "12 to 18 (161 mm to 230 mm)", "A long airbag strap is applicable to this range" is displayed at the bottom of the interface. Certainly, in another embodiment, the electronic device 100 may also automatically measure a wrist of the user to obtain wrist circumference data of the user. Alternatively, the wrist circumference data may be input in a process of identifying the strap 20.

S320: Obtain a magnetic pole of the strap 20.

Specifically, as shown in FIG. 5 and FIG. 23, the electronic device 100 includes a watch body 10 and the strap 20. An airbag 50 is disposed on an inner side of the strap 20. In this embodiment, there are three types of straps 20: a short airbag strap, a long airbag strap, and a common strap. Airbags are disposed on both the short airbag strap and the long airbag strap, and an airbag is not disposed on the common strap. A first airbag is disposed on the short airbag strap, and a second airbag is disposed on the long airbag strap. A length of the first airbag is different from a length of the second airbag, and the common strap includes a cover. The short airbag strap is a first strap, and the long airbag strap and the common strap are second straps.

During blood pressure measurement, users with different wrist circumferences may select straps 20 with different airbag lengths as required. On the premise that an airbag of the strap 20 can fully cover a radial artery and an ulnar artery, it can be further ensured that a length of the strap 20 is not excessively long, thereby ensuring user experience. When there is no need for blood pressure measurement, the user may select the common strap, to improve wearing comfort.

A Hall effect sensor 16 is disposed on the watch body 10, and a magnet is disposed on the airbag 50 or the cover of the strap 20. Magnets are disposed on the airbags or covers of the first strap and the second strap in different manners. When the strap 20 is the first strap, the strap 20 is disposed on the watch body 10, and a first magnetic pole of a first magnet is disposed opposite to the Hall effect sensor 16 of the watch body 10. When the strap 20 is the second strap, the strap 20 is disposed on the watch body 10, and a second magnetic pole of a second magnet is disposed opposite to the Hall effect sensor 16 of the watch body 10. The first magnetic pole is different from the second magnetic pole. The first magnetic pole is an S pole, and the second magnetic pole is an N pole. Because the Hall effect sensor 16 can detect a magnetic field change, and magnetic poles of different straps 20 are different, the watch body 10 can identify a magnetic pole to obtain whether the strap 20 is the first strap or the second strap.

Before the magnetic pole of the strap 20 is obtained, the method further includes: receiving a strap 20 identification instruction. It may be understood that when a processor 13 detects some operations of the user, the processor 13 may send the strap 20 identification instruction to the Hall effect sensor 16. For example, after the processor 13 detects that the user enters the wrist circumference data and taps "Next", the processor 13 sends the strap 20 identification instruction to the Hall effect sensor 16. Alternatively, when the processor 13 detects that the user starts the electronic device 100 for the first time, or when the processor 13 detects that the user opens a blood pressure measurement function page of the electronic device 100, the processor 13 sends the strap 20 identification instruction to the Hall effect sensor 16. Alternatively, when the processor 13 detects that the user disposes the strap 20 on the watch body 10, the processor 13 sends the strap 20 identification instruction to the Hall effect sensor 16.

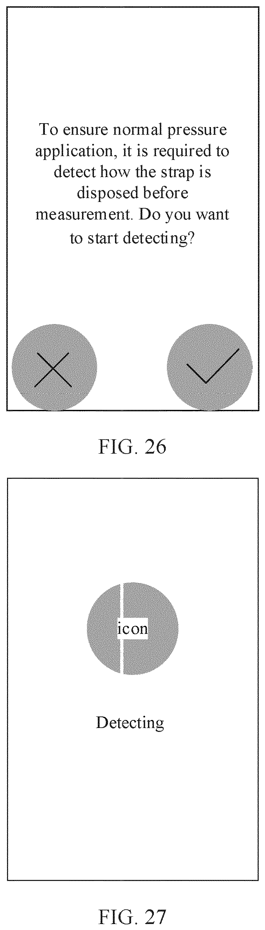

As shown in FIG. 26 and FIG. 27, after receiving the strap 20 identification instruction, the Hall effect sensor 16 may send, to the user interface, information about whether to start detection. The user interface displays "To ensure normal pressure application, it is required to detect how the strap is disposed before measurement. Do you want to start detecting?". If the user confirms to perform detection, the user taps "√". If the user does not perform detection, the user taps " ×". When the user taps "√", "Detecting" is displayed on the user interface.

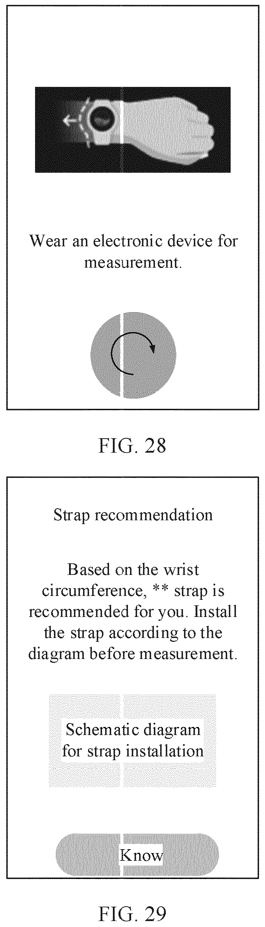

During the detection, the electronic device 100 first detects whether the user wears the electronic device 100. If the user does not wear the electronic device 100, as shown in FIG. 28, the user interface displays "Wear an electronic device for measurement" and a figure to prompt the user to wear the electronic device 100. If the user wears the electronic device 100, the magnetic pole of the strap 20 is detected. Specifically, the processor 13 sends the strap 20 identification instruction to the Hall effect sensor 16. After receiving the strap 20 identification instruction, the Hall effect sensor 16 detects the magnetic pole of the strap 20, to obtain the magnetic pole of the strap 20.

S330: Determine, based on the magnetic pole of the strap 20, that the strap 20 is the first strap or the second strap.

Specifically, as shown in FIG. 23, if the Hall effect sensor detects a magnetic field of the first magnetic pole, the strap 20 is the first strap, to be specific, the strap 20 is the short airbag strap. If the Hall effect sensor detects a magnetic field of the second magnetic pole, the strap 20 is the second strap. The first strap is applicable to a user with a small wrist circumference for blood pressure measurement. For example, the first strap may be applicable to a user with a wrist circumference ranging from 130 mm to 160 mm (including 130 mm and 160 mm).

Certainly, if the magnetic pole of the strap 20 is no magnetic pole, the airbag or the cover is not disposed on the watch body 10, or the airbag or the cover is loose from the watch body 10.

S340: If the strap 20 is the second strap, inflate the strap 20, to obtain a pressurization curve slope of the strap 20.

Specifically, as shown in FIG. 23, if the strap 20 is the second strap, it indicates that the second strap is the long airbag strap or the common strap. It may be understood that the air pump 141 in the watch body 10 may inflate the second strap. Because the long airbag strap has an airbag, and the common strap does not have an airbag, a pressure sensor 142 identifies different atmospheric pressure changes when the long airbag strap and the common strap are in an inflated state. Because the common strap does not have the airbag and cannot store air, a pressurization curve slope of the common strap increases rapidly in a short time according to a fixed slope. The long airbag strap has the airbag, and a pressurization curve slope of the long airbag strap increases slowly in a period of time according to a fixed slope. Pressurization curve slopes of the long airbag strap and the common strap in the inflated state are different. The pressurization curve slope is test data. Test data of the second strap is compared with preset data (the pressurization curve slope of the long airbag strap and the pressurization curve slope of the common strap).

**If** the pressurization curve slope of the second strap matches the pressurization curve slope of the long airbag strap, it indicates that the test data is first data, and the second strap is the long airbag strap. The second strap is applicable to a user with a large wrist circumference. For example, the second strap may be applicable to a user with a wrist circumference ranging from 161 mm to 230 mm (including 161 mm and 230 mm). If the pressurization curve slope of the second strap matches the pressurization curve slope of the common strap, it indicates that the test data is second data, and the second strap is the common strap. The common strap is applicable to wearing when the user does not measure blood pressure, thereby improving user comfort.

Certainly, in another embodiment, when the Hall effect sensor 16 identifies the magnetic pole of the strap 20, the air pump 141 in the watch body 10 may inflate the strap 20. In other words, step 330 and step 340 may be performed simultaneously, to determine, in a short time, whether the strap 20 is the common strap. Therefore, the processor 13 may quickly determine a type of the strap 20 in a short time based on a joint detection result of how the Hall effect sensor 16 and the magnetic pole collaborate and how to inflate the strap 20.

Alternatively, when the processor 13 identifies that the wrist circumference data of the user corresponds to the long airbag strap, a controller may control the Hall effect sensor 16 to identify the magnetic pole of the strap 20 and control the air pump 141 in the watch body 10 to inflate the strap 20 to identify the strap 20. It may be understood that step 330 and step 340 are performed simultaneously, to determine, in a short time, whether the strap 20 is the common strap. Therefore, the processor 13 may quickly determine the type of the strap 20 in a short time based on a joint detection result of how the Hall effect sensor 16 and the magnetic pole collaborate and how to inflate the strap 20.

S350: Compare the type of the strap 20 with the wrist circumference data of the user, and send a comparison result to the user interface.

Specifically, as shown in FIG. 23, when it is detected in step S340 that the strap 20 is the common strap, as shown in FIG. 29, it is detected that the strap 20 with an airbag is not disposed. A user interface of strap 20 recommendation is displayed. The user interface displays "Based on the wrist circumference, a short airbag strap (or a long airbag strap) is recommended for you. Install the strap according to the diagram before measurement." The user interface displays a schematic diagram to install the strap 20.

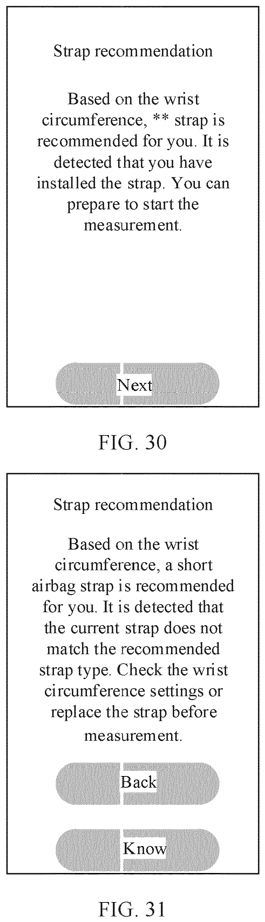

When it is detected in step S330 and step S340 that the strap 20 is the long airbag strap, the long airbag strap is compared with the wrist circumference data, to determine whether the strap 20 corresponding to the wrist circumference data is the long airbag strap. As shown in FIG. 30, if the strap 20 corresponding to the wrist circumference data is the long airbag strap, the user interface displays "Based on the wrist circumference, a long airbag strap is recommended for you. It is detected that you have installed the strap. You can start measuring blood pressure". If the user wants to measure blood pressure, the user taps "Next" to start measuring blood pressure. As shown in FIG. 31, if the strap 20 corresponding to the wrist circumference data is not the long airbag strap, the user interface displays "Based on the wrist circumference, a short airbag strap is recommended for you. It is detected that the current strap does not match the recommended strap type. Check the wrist circumference settings or replace the strap before measurement." The user may tap "Back" or "Know" as required.

When it is detected in step S330 and step S340 that the strap 20 is the short airbag strap, the short airbag strap is compared with the wrist circumference data, to determine whether the strap 20 corresponding to the wrist circumference data is the short airbag strap. If the strap 20 corresponding to the wrist circumference data is the short airbag strap, the user interface displays "Based on the wrist circumference, a short airbag strap is recommended for you. It is detected that you have installed the strap. You can start measuring blood pressure". If the user wants to measure blood pressure, the user taps "Next" to start measuring blood pressure. If the strap 20 corresponding to the wrist circumference data is not the short airbag strap, the user interface displays "Based on the wrist circumference, a long airbag strap is recommended for you. It is detected that the current strap does not match the recommended strap type. Check the wrist circumference settings or replace the strap before measurement." The user may tap "Back" or "Know" as required.

In this embodiment, the type of the strap 20 disposed on the watch body 10 is identified, an identification result is compared with the wrist circumference of the user, and a comparison result is sent to the user interface. The user may learn, based on the identification result, whether the user wears an incorrect strap, and may replace the strap 20 or perform a subsequent operation under a prompt, to avoid a problem that blood pressure precision decreases because the user uses the incorrect strap. In addition, if the user receives that the strap 20 is not disposed on the watch body 10 of the electronic device 100, the user may dispose the strap 20 on the watch body 10 or check whether the strap 20 is loose, to avoid that the user washes hands or takes a shower to cause water damage to the device when the strap 20 is loose.

It may be understood that the electronic device 100 may support storage of wrist circumference data of a plurality of users. When the electronic device 100 stores wrist circumference data of one user, in step 350, the wrist circumference data of the user is directly invoked for comparison. When the electronic device 100 stores the wrist circumference data of the plurality of users, in a process of identifying the strap 20, the user may select the wrist circumference data of the user for comparison.

Certainly, in another embodiment, the electronic device 100 may alternatively identify the type of the strap 20 without using collaboration between the Hall effect sensor 16 and the magnet 54. For example, the type of the strap 20 may be identified by inflating the strap 20. Specifically, after receiving the strap 20 identification instruction, the processor 13 controls an air pump 141 to inflate the strap 20, and a pressure sensor 142 obtains an atmospheric pressure change value, to obtain a pressurization curve slope of the strap 20 in an inflated state. It may be understood that, airbag lengths of the long airbag strap and the short airbag strap are different, and the common strap does not have the airbag. Therefore, pressurization curve slopes of the long airbag strap, the short airbag strap, and the common strap are different, and the pressurization curve slopes of the long airbag strap, the short airbag strap, and the common strap may be stored in the processor 13 in advance. The obtained pressurization curve slope is compared with a pre-stored pressurization curve slope, to determine whether the strap 20 is the long airbag strap, the short airbag strap, or the common strap.

The foregoing descriptions are merely some embodiments and implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. An electronic device, comprising
a watch body, wherein the watch body(10) comprises an air pump(141), a pressure sensor(142), a Hall effect sensor(16) and a processor(13);
a first strap with a first airbag, wherein the first airbag is detachably connected to the watch body(10) by using a first mounting part of the first strap, a first magnet is disposed on the first mounting part, and a first magnetic pole of the first magnet is disposed close to the first mounting part;
a second strap with a second airbag, wherein the second airbag is detachably connected to the watch body(10) by using a second mounting part of the second strap, a second magnet is disposed on the second mounting part, and
a second magnetic pole of the second magnet is disposed close to the second mounting part; and
the second magnetic pole is different from the first magnetic pole, a length of the first airbag is different from a length of the second airbag; and
wherein the processor(13) is configured to:
if the Hall effect sensor detects a magnetic field of the first magnetic pole, determine that the first strap is disposed on the watch body; or
if the Hall effect sensor detects a magnetic field of the second magnetic pole, determine that the second strap is disposed on the watch body;
further control the air pump to inflate the first strap or the second strap, to obtain test data, and detect that the first strap or the second strap is disposed on the watch body based on the test data; and
if the Hall effect sensor detects that there is no magnetic field, determine that an airbag is not disposed on the watch body.

2. The electronic device according to claim 1, wherein the first magnetic pole is an S pole, and the second magnetic pole is an N pole.

3. The electronic device according to claim 1, wherein the first magnetic pole is an N pole, and the second magnetic pole is an S pole.

4. The electronic device according to any one of claims 1 to 3, wherein the electronic device further comprises an obtaining module and a comparison module, the obtaining module configured to obtain wrist circumference data of a user, and compare the wrist circumference data with a type of strap, to determine that the type of strap, is selected correctly or incorrectly.

5. The electronic device according to claim 4, wherein the processor(13) is further configured to: if the strap is selected incorrectly, remind the user to replace strap with a correct type.

6. The electronic device according to claim 4 or 5, wherein the watch body(10) further comprises a touchscreen(12) or a button, the touchscreen or the button is configured to receive an input operation of the user, and the processor(13) is configured to obtain an electrical signal of the touchscreen(12) or the button, to obtain the wrist circumference data input by the user.

7. The electronic device according to claim 1, wherein the second strap comprises a first strap body and a second strap body, the first strap body and the second strap body are respectively connected to two sides of the watch body(10), the second airbag is disposed on an inner side of the first strap body, and the second magnet is disposed on a part that is of the second airbag and that is close to the watch body(10).

8. The electronic device according to claim 1, wherein the second strap comprises a first strap body and a second strap body, the first strap body and the second strap body are respectively connected to two sides of the watch body(10), the second airbag is disposed on inner sides of the first strap body and the watch body(10), and the second magnet is disposed on a part that is of the second airbag and that is close to the watch body(10).

9. The electronic device according to claim 1, wherein the second strap comprises a first strap body and a second strap body, the first strap body and the second strap body are respectively connected to two sides of the watch body(10), the second airbag is disposed on inner sides of the first strap body, the watch body(10), and the second strap body, and the second magnet is disposed on a part that is of the second airbag and that is close to the watch body(10).

10. The electronic device according to any one of claims 1 to 10, wherein the first strap with the first airbag is applicable to a user whose wrist circumference ranges from 130 mm to 160 mm, and the second strap with the second airbag is applicable to a user whose wrist circumference ranges from 161 mm to 230 mm.

## Patentansprüche

1. Elektronische Vorrichtung, umfassend:
ein Uhrengehäuse, wobei das Uhrengehäuse (10) eine Luftpumpe (141), einen Drucksensor (142), einen Hall-Effekt-Sensor (16) und einen Prozessor (13) umfasst;
ein erstes Armband mit einem ersten Airbag, wobei der erste Airbag unter Verwendung eines ersten Befestigungsteils des ersten Armbands lösbar mit dem Uhrengehäuse (10) verbunden ist, ein erster Magnet auf dem ersten Befestigungsteil angeordnet ist und ein erster Magnetpol des ersten Magneten in der Nähe des ersten Befestigungsteils angeordnet ist;
ein zweites Armband mit einem zweiten Airbag, wobei der zweite Airbag unter Verwendung eines zweiten Befestigungsteils des zweiten Armbands lösbar mit dem Uhrengehäuse (10) verbunden ist, ein zweiter Magnet auf dem zweiten Befestigungsteil angeordnet ist und ein zweiter Magnetpol des zweiten Magneten in der Nähe des zweiten Befestigungsteils angeordnet ist; und
sich der zweite Magnetpol von dem ersten Magnetpol unterscheidet, sich eine Länge des ersten Airbags von einer Länge des zweiten Airbags unterscheidet; und
wobei der Prozessor (13) dazu konfiguriert ist:
wenn der Hall-Effekt-Sensor ein Magnetfeld des ersten Magnetpols erkennt, zu bestimmen, dass das erste Armband an dem Uhrengehäuse angeordnet ist; oder
wenn der Hall-Effekt-Sensor ein Magnetfeld des zweiten Magnetpols erkennt, zu bestimmen, dass das zweite Armband an dem Uhrengehäuse angeordnet ist;
ferner die Luftpumpe zum Aufpumpen des ersten Armbands oder des zweiten Armbands zu steuern, um Testdaten zu erfassen, und basierend auf den Testdaten zu erkennen, dass das erste Armband oder das zweite Armband an dem Uhrengehäuse angeordnet ist; und
wenn der Hall-Effekt-Sensor erkennt, dass kein Magnetfeld vorhanden ist, zu bestimmen, dass kein Airbag an dem Uhrengehäuse angeordnet ist.

2. Elektronische Vorrichtung nach Anspruch 1, wobei der erste Magnetpol ein S-Pol ist und der zweite Magnetpol ein N-Pol ist.

3. Elektronische Vorrichtung nach Anspruch 1, wobei der erste Magnetpol ein N-Pol ist und der zweite Magnetpol ein S-Pol ist.

4. Elektronische Vorrichtung nach einem der Ansprüche 1 bis 3, wobei die elektronische Vorrichtung ferner ein Erfassungsmodul und ein Vergleichsmodul umfasst, wobei das Erfassungsmodul dazu konfiguriert ist, Handgelenksumfangsdaten eines Benutzers zu erfassen und die Handgelenksumfangsdaten mit einem Armbandtyp zu vergleichen, um zu bestimmen, dass der Armbandtyp richtig oder falsch ausgewählt ist.

5. Elektronische Vorrichtung nach Anspruch 4, wobei der Prozessor (13) ferner dazu konfiguriert ist: wenn das Armband falsch ausgewählt ist, den Benutzer daran zu erinnern, das Armband durch einen richtigen Typ zu ersetzen.

6. Elektronische Vorrichtung nach Anspruch 4 oder 5, wobei das Uhrengehäuse (10) ferner einen Touchscreen (12) oder einen Knopf umfasst, der Touchscreen oder der Knopf dazu konfiguriert ist, eine Eingabeoperation des Benutzers zu empfangen, und der Prozessor (13) dazu konfiguriert ist, ein elektrisches Signal des Touchscreens (12) oder des Knopfes zu erfassen, um die durch den Benutzer eingegebenen Handgelenkumfangsdaten zu erfassen.

7. Elektronische Vorrichtung nach Anspruch 1, wobei das zweite Armband einen ersten Armbandkörper und einen zweiten Armbandkörper umfasst, der erste Armbandkörper und der zweite Armbandkörper jeweils mit zwei Seiten des Uhrengehäuses (10) verbunden sind, der zweite Airbag auf einer Innenseite des ersten Armbandkörpers angeordnet ist und der zweite Magnet an einem Teil angeordnet ist, der von dem zweiten Airbag stammt und der sich in der Nähe des Uhrengehäuses (10) befindet.

8. Elektronische Vorrichtung nach Anspruch 1, wobei das zweite Armband einen ersten Armbandkörper und einen zweiten Armbandkörper umfasst, der erste Armbandkörper und der zweite Armbandkörper jeweils mit zwei Seiten des Uhrengehäuses (10) verbunden sind, der zweite Airbag auf Innenseiten des ersten Armbandkörpers und des Uhrengehäuses (10) angeordnet ist, und der zweite Magnet an einem Teil angeordnet ist, der von dem zweiten Airbag stammt und der sich in der Nähe des Uhrengehäuses (10) befindet.

9. Elektronische Vorrichtung nach Anspruch 1, wobei das zweite Armband einen ersten Armbandkörper und einen zweiten Armbandkörper umfasst, der erste Armbandkörper und der zweite Armbandkörper jeweils mit zwei Seiten des Uhrengehäuses (10) verbunden sind, der zweite Airbag auf Innenseiten des ersten Armbandkörpers, des Uhrengehäuses (10) und des zweiten Armbandkörpers angeordnet ist, und der zweite Magnet an einem Teil angeordnet ist, der von dem zweiten Airbag stammt und der sich in der Nähe des Uhrengehäuses (10) befindet.

10. Elektronische Vorrichtung nach einem der Ansprüche 1 bis 10, wobei das erste Armband mit dem ersten Airbag auf einen Benutzer anwendbar ist, dessen Handgelenkumfang zwischen 130 mm und 160 mm liegt, und das zweite Armband mit dem zweiten Airbag auf einen Benutzer anwendbar ist, dessen Handgelenkumfang zwischen 161 mm und 230 mm liegt.

## Revendications

1. Dispositif électronique, comprenant
un corps de montre, dans lequel le corps de montre (10) comprend une pompe à air (141), un capteur de pression (142), un capteur à effet Hall (16) et un processeur (13) ;
un premier bracelet avec un premier airbag, dans lequel le premier airbag est relié de manière détachable au corps de montre (10) en utilisant une première partie de montage du premier bracelet, un premier aimant est disposé sur la première partie de montage, et un premier pôle magnétique du premier aimant est disposé près de la première partie de montage ;
un second bracelet avec un second airbag, dans lequel le second airbag est relié de manière détachable au corps de montre (10) en utilisant une seconde partie de montage du second bracelet,
un second aimant est disposé sur la seconde partie de montage, et
un second pôle magnétique du second aimant est disposé près de la seconde partie de montage ; et
le second pôle magnétique est différent du premier pôle magnétique, une longueur du premier airbag est différente d'une longueur du second airbag; et
dans lequel le processeur (13) est configuré pour :
si le capteur à effet Hall détecte un champ magnétique du premier pôle magnétique, déterminer que le premier bracelet est disposé sur le corps de montre ; ou
si le capteur à effet Hall détecte un champ magnétique du second pôle magnétique, déterminer que le second bracelet est disposé sur le corps de montre ;
commander également la pompe à air pour gonfler le premier bracelet ou le second bracelet, pour obtenir des données de test, et détecter que le premier bracelet ou le second bracelet est disposé sur le corps de montre sur la base des données de test ; et
si le capteur à effet Hall détecte qu'il n'y a pas de champ magnétique, déterminer qu'un airbag n'est pas disposé sur le corps de montre.

2. Dispositif électronique selon la revendication 1, dans lequel le premier pôle magnétique est un pôle S, et le second pôle magnétique est un pôle N.

3. Dispositif électronique selon la revendication 1, dans lequel le premier pôle magnétique est un pôle N, et le second pôle magnétique est un pôle S.

4. Dispositif électronique selon l'une quelconque des revendications 1 à 3, dans lequel le dispositif électronique comprend également un module d'obtention et un module de comparaison, le module d'obtention étant configuré pour obtenir des données de circonférence de poignet d'un utilisateur, et comparer les données de circonférence de poignet avec un type de bracelet, pour déterminer que le type de bracelet, est sélectionné correctement ou incorrectement.

5. Dispositif électronique selon la revendication 4, dans lequel le processeur (13) est également configuré pour : si le bracelet est sélectionné incorrectement, rappeler à l'utilisateur de remplacer le bracelet par un type correct.

6. Dispositif électronique selon la revendication 4 ou 5, dans lequel le corps de montre (10) comprend également un écran tactile (12) ou un bouton, l'écran tactile ou le bouton est configuré pour recevoir une opération d'entrée de l'utilisateur, et le processeur (13) est configuré pour obtenir un signal électrique de l'écran tactile (12) ou du bouton, pour obtenir les données de circonférence de poignet entrées par l'utilisateur.

7. Dispositif électronique selon la revendication 1, dans lequel le second bracelet comprend un premier corps de bracelet et un second corps de bracelet, le premier corps de bracelet et le second corps de bracelet sont respectivement reliés à deux côtés du corps de montre (10), le second airbag est disposé sur un côté intérieur du premier corps de bracelet, et le second aimant est disposé sur une partie qui est du second airbag et qui est proche du corps de montre (10).

8. Dispositif électronique selon la revendication 1, dans lequel le second bracelet comprend un premier corps de bracelet et un second corps de bracelet, le premier corps de bracelet et le second corps de bracelet sont respectivement reliés à deux côtés du corps de montre (10), le second airbag est disposé sur des faces intérieures du premier corps de bracelet et du corps de montre (10), et le second aimant est disposé sur une partie qui est du second airbag et qui est proche du corps de montre (10).

9. Dispositif électronique selon la revendication 1, dans lequel le second bracelet comprend un premier corps de bracelet et un second corps de bracelet, le premier corps de bracelet et le second corps de bracelet sont respectivement reliés à deux côtés du corps de montre (10), le second airbag est disposé sur des côtés intérieurs du premier corps de bracelet, du corps de montre (10) et du second corps de bracelet, et le second aimant est disposé sur une partie qui est du second airbag et qui est proche du corps de montre (10).

10. Dispositif électronique selon l'une quelconque des revendications 1 à 10, dans lequel le premier bracelet avec le premier airbag est applicable à un utilisateur dont la circonférence de poignet est comprise entre 130 mm et 160 mm, et le second bracelet avec le second airbag est applicable à un utilisateur dont la circonférence de poignet est comprise entre 161 mm et 230 mm.
